# EUROPÄISCHE PATENTSCHRIFT

(11) **EP 3 419 702 B1**
(45) Veröffentlichungstag und Bekanntmachung des Hinweises auf die Patenterteilung: **22.02.2023**
(21) Anmeldenummer: 17705291.7
(22) Anmeldetag: 02.02.2017
(51) Int. Cl.: A61M 5/315, A61M 5/31, A61M 5/32

(54) **INJEKTIONSVORRICHTUNG MIT EINEM SEITLICH ANGEBRACHTEN BETÄTIGUNGSGLIED ZUR AUSLÖSUNG DER PRODUKTAUSSCHÜTTUNG**
INJECTION DEVICE WITH LATERALLY ARRANGED ACTUATION MEMBER FOR TRIGGERING PRODUCT DELIVERY
APPAREIL D'INJECTION AVEC ÉLÉMENT D'ACTIONNEMENT DISPOSÉ LATÉRALEMENT POUR DÉCLENCHER L'ADMINISTRATION D'UN PRODUIT

(30) Priorität: 23.02.2016 CH 2362016
(43) Veröffentlichungstag der Anmeldung: 02.01.2019
(73) Patentinhaber: Ypsomed AG, 3401 Burgdorf (CH)
(72) Erfinder: STREIT, Ursina, 3322 Schönbühl (CH); SCHENKER, Susanne, 4912 Aarwangen (CH); HIRSCHEL, Jürg, 3007 Bern (CH)
(74) Vertreter: Meier Obertüfer, Jürg
(86) Internationale Anmeldenummer: PCT/CH2017/000010
(87) Internationale Veröffentlichungsnummer: WO 2017/143462

(56) Entgegenhaltungen:
- WO-A1-02/053214
- WO-A1-2007/017052
- WO-A1-2015/032771
- WO-A2-2014/166922
- US-A1- 2012 136 306

## Beschreibung

Die Erfindung betrifft eine Injektionsvorrichtung zum Verabreichen eines flüssigen Produkts, insbesondere eines Medikaments, wie zum Beispiel Insulin zur Diabetestherapie. Im Besonderen betrifft die Erfindung eine Antriebs- und Dosiervorrichtung für eine solche Injektionsvorrichtung. Die Antriebs- und Dosiervorrichtung weist ein Betätigungsglied mit einem Betätigungsabschnitt auf, der seitlich an der länglichen Antriebs- und Dosiervorrichtung oder Injektionsvorrichtung angebracht ist und der nicht das hintere Ende der Vorrichtung bildet.

Der Begriff "Medikament" umfasst hier jede fließfähige medizinische Formulierung, welche geeignet ist zur kontrollierten Verabreichung durch ein Mittel, wie zum Beispiel eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (zum Beispiel Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfsund Trägerstoffe.

Aus dem Stand der Technik, wie zum Beispiel der WO 2013/144020 A1, der EP 2 829 292 A1 und der WO 2015/081451 A1 sind Injektionsvorrichtungen bekannt, die an ihren proximalen Enden einen Betätigungsknopf aufweisen, dessen Betätigung die Ausschüttung einer zuvor eingestellten Produktdosis auslöst. Aus der US 5,104,380 A1, der WO 02/053214 A1 und der WO 2007/017052 A1 sind Injektionsvorrichtungen bekannt, die ein seitlich an der Injektionsvorrichtung angebrachtes Auslöseglied aufweisen, dessen Betätigung eine Produktausschüttung auslöst.

Es ist eine Aufgabe der Erfindung, eine Antriebs- und Dosiervorrichtung für eine Injektionsvorrichtung bereitzustellen, wobei die Antriebs- und Dosiervorrichtung einen hohen Bedienkomfort bieten und einfach in der Herstellung sein soll.

Die Aufgabe wird mit den Merkmalen des Anspruchs 1 gelöst. Vorteilhafte Weiterentwicklungen ergeben sich aus den abhängigen Ansprüchen, der Beschreibung und den Figuren.

Die Erfindung geht von einer Antriebs- und Dosiervorrichtung für eine Injektionsvorrichtung zum Verabreichen eines flüssigen Medikaments oder Produkts aus. Die Antriebs- und Dosiervorrichtung weist ein Gehäuse auf. Das Gehäuse ist vorzugsweise hülsenförmig und/oder länglich ausgebildet. Das Gehäuse kann sich zum Beispiel entlang seiner Längsachse bzw. der Längsachse der Antriebs- und Dosiervorrichtung erstrecken.

Das Gehäuse kann optional einen Produktbehälter aufnehmen oder selbst den Produktbehälter bilden. Das Gehäuse kann ein- oder mehrteilig sein. Zum Beispiel kann das Gehäuse einen proximalen Gehäuseteil bilden, der im Wesentlichen die Antriebs- und Dosiervorrichtung bildet, d. h. einen Antriebs- und Dosiermechanismus umfasst oder aufweist. Das Gehäuse kann ferner einen Produktbehälterhalter aufweisen, der den Produktbehälter, wie zum Beispiel eine Karpule, aufnimmt und mit dem Gehäuse oder dem proximalen Gehäuseteil verbunden ist. Diese Verbindung kann derart sein, dass der Produktbehälterhalter und das Gehäuse oder das proximale Gehäuseteil nach dem Verbinden unlösbar, d. h. nur noch durch Zerstörung von Verbindungselementen lösbar ist. Eine solche Ausführung ist insbesondere bei Einweginjektionsvorrichtungen von Vorteil, die nach dem das in dem Produktbehälter enthaltene Produkt vollständig ausgeschüttet ist, als Ganzes entsorgt werden. Alternativ kann der Produktbehälterhalter auch lösbar an dem Gehäuse befestigt werden, wodurch es möglich ist, die Antriebs- und Dosiervorrichtung ggf. mehrfach zu verwenden, d. h. einen leeren Produktbehälter gegen einen gefüllten Produktbehälter auszutauschen.

Das Gehäuse dient vorzugsweise dazu, vom Benutzer der Vorrichtung ergriffen zu werden. Insbesondere kann das Gehäuse eine im Wesentlichen zylindrische Form aufweisen.

Die Antriebs- und Dosiervorrichtung umfasst ein Dosiseinstellglied, das zum Beispiel als Dosierknopf ausgebildet ist. Das Dosiseinstellglied ist vorzugsweise vom Benutzer (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Für die Einstellung der auszuschüttenden oder zu verabreichenden Dosis wird das Dosiseinstellglied vorzugsweise vom Benutzer ergriffen und relativ zu dem Gehäuse um eine Drehachse, die vorzugsweise der Längsachse der zum Beispiel länglich ausgestalteten Antriebs- und Dosiervorrichtung entspricht, verdreht. Das zum Beispiel hülsenförmige Dosiseinstellglied kann vorzugsweise axialfest mit dem Gehäuse verbunden, insbesondere entlang der Längsachse des Gehäuses verschiebefest sein, wodurch vorteilhaft die intuitive Handhabung der Vorrichtung durch den Benutzer erleichtert wird, da er lediglich eine Drehbewegung des Dosiseinstellglieds für die Dosiseinstellung auszuführen braucht.

Das Dosiseinstellglied kann zum Beispiel an dem hinteren Ende des Gehäuses oder der Antriebs- und Dosiervorrichtung angeordnet sein. Insbesondere kann das Dosiseinstellglied das hintere Ende der Antriebs- und Dosiervorrichtung bilden. Im Vergleich dazu, werden bei der WO 2013/144020 A1, EP 2 829 292 A1 und WO 2015/081451 A1 das hintere Ende der Antriebs- und Dosiervorrichtung von einem Betätigungsglied gebildet. Das für die erfindungsgemäße Antriebs- und Dosiervorrichtung vorgesehene Dosiseinstellglied kann an seinem hinteren, d. h. proximalen Ende mittels einer Stirnwand verschlossen sein.

Die Antriebs- und Dosiervorrichtung weist ein Betätigungsglied auf, welches zum Beispiel hülsen- oder schalenförmig (Hülsensegment) sein kann. Das Betätigungsglied dient dazu, die Ausschüttung der mit dem Dosiseinstellglied eingestellten Produktdosis auszulösen. Hierfür ist das Betätigungsglied aus einer unbetätigten Position, die es während der Einstellung der auszuschüttenden Produktdosis einnimmt in eine betätigte Position, die es während der Produktausschüttung einnimmt, verschiebbar. Mit anderen Worten, bewirkt die Verschiebung des Betätigungsglieds aus der unbetätigten Position in die betätigte Position das Auslösen der Produktausschüttung. Das Betätigungsglied ist manuell, d. h. von Hand betätigbar, d. h. zumindest aus der unbetätigten Position in die betätigte Position verschiebbar, beispielsweise auch aus der betätigten Position in die unbetätigte Position. Alternativ kann die Bewegung aus der betätigten Position in die unbetätigte Position von einer mittelbar oder unmittelbar auf das Betätigungsglied wirkenden Rücksetzfeder übernommen oder unterstützt werden. Die Rücksetzfeder und das Betätigungsglied können so, insbesondere unmittelbar oder mittelbar miteinander verbunden sein, dass eine Verschiebung des Betätigungsglieds aus der unbetätigten Position ein Spannen der Rücksetzfeder bewirkt und eine Bewegung des Betätigungsglieds aus der betätigten Position in die unbetätigte Position ein Entspannen der Feder bewirkt.

Das Betätigungsglied ist so ausgestaltet und angeordnet, dass ein Benutzer der Antriebs- und Dosiervorrichtung Zugriff darauf hat. Somit kann das Betätigungsglied eine äußere Oberfläche der Antriebs- und Dosiervorrichtung bilden und/oder von außen zugänglich sein, zumindest ein Abschnitt davon. Insbesondere kann zumindest ein von dem Betätigungsglied gebildeter Betätigungsabschnitt so ausgebildet und angeordnet sein, dass der Benutzer der Antriebs- und Dosiervorrichtung Zugriff darauf hat, wobei insbesondere durch Manipulation oder Betätigung des Betätigungsabschnitts das Betätigungsglied aus der unbetätigten in die betätigte Position verschiebbar ist. Zum Beispiel kann der Betätigungsabschnitt so angeordnet sein, dass der Benutzer der Antriebs- und Dosiervorrichtung den Betätigungsabschnitt mit mindestens einem Glied, insbesondere mindestens einem Finger, der Hand, welche das Gehäuse der Antriebs- und Dosiervorrichtung umgreift aus der unbetätigten Position in die betätigte Position verschieben kann. Beispielsweise kann der Betätigungsabschnitt einstückig oder einteilig von dem Betätigungsglied gebildet sein. Allgemein kann mittels des Betätigungsabschnitts der Benutzer das Betätigungsglied aus der unbetätigten Position in die betätigte Position verschieben.

Beispielsweise lässt sich das Betätigungsglied vorteilhaft mit dem Daumen der Hand, die das Gehäuse umgreift, betätigen, insbesondere drücken. Durch Loslassen des Betätigungsglieds kann die Betätigung beendet werden. Unter "betätigen" wird die Verschiebung des Betätigungsglieds insbesondere entlang der Längsachse der Antriebs- und Dosiervorrichtung, wie zum Beispiel in die distale Richtung verstanden, wodurch die Produktausschüttung ausgelöst werden kann. Das Betätigungsglied ist vorteilhaft relativ zu dem Gehäuse entlang der Längsachse verschiebbar und kann zum Beispiel von dem Gehäuse axial verschiebbar geführt oder aufgenommen sein.

Das Betätigungsglied kann vorteilhaft gegen die Kraft der Rücksetz- oder Betätigungsfeder betätigbar sein, wobei diese Feder so auf das Betätigungsglied wirkt, dass sie gespannt wird, wenn das Betätigungsglied aus seiner unbetätigten Position in seine betätigte Position verschoben wird. Mittels der Rücksetzfeder wird das Betätigungsglied aus seiner betätigten Position in seine unbetätigte Position zurückgesetzt oder verschoben, insbesondere mit einer Bewegung in die proximale Richtung relativ zu dem Gehäuse. Das Rücksetzen des Betätigungsglieds durch die Feder kann zum Beispiel dadurch erfolgen, dass der Benutzer den Betätigungsabschnitt los lässt.

Der Betätigungsabschnitt kann distal des Dosiseinstellglieds, welches zum Beispiel das proximale, d. h. das Ende, welches dem distalen Ende, an dem sich die Nadel befindet, gegenüberliegt, bildet, angeordnet sein. Demnach ist das Dosiseinstellglied proximal des Betätigungsglieds, insbesondere des Betätigungsabschnitts angeordnet. Das Betätigungsglied, insbesondere dessen Betätigungsabschnitt kann zwischen dem distalen Ende und dem proximalen Ende der Antriebs- und Dosiervorrichtung angeordnet sein, insbesondere zwischen dem distalen Ende und dem Dosiseinstellglied.

Die Antriebs- und Dosiervorrichtung kann ein Dosisanzeigeglied, wie zum Beispiel eine Dosisanzeigetrommel oder mindestens einen Dosiszählring, aufweisen. Das Dosisanzeigeglied weist an seinem Außenumfang eine über den Umfang umlaufende Skala auf, die Skalenwerte aufweist, die die eingestellte Produktdosis wiedergeben. Die Skala kann bei einem Dosisanzeigering über den Umfang umlaufen, wobei die Dosisskala bei einer Dosisanzeigetrommel insbesondere wendel- oder helixförmig über den Umfang umlaufen kann.

Die Antriebs- und Dosiervorrichtung kann eine Zeigeeinrichtung aufweisen, wie zum Beispiel ein Fenster, das zum Beispiel als Durchbruch oder aus einem transparenten Material gebildet sein kann. Die Zeigeeinrichtung kann so ausgebildet und angeordnet sein, dass durch sie ein Skalenwert der Dosisskala, welcher mit der eingestellten Dosis korrespondiert, abgelesen werden kann. Das Dosisanzeigeglied kann zum Beispiel im Querschnitt ringförmig sein. Das Dosisanzeigeglied kann zum Beispiel eine Dosisanzeigetrommel oder ein Dosisanzeigering sein. Die Dosisskala kann sich über den Umfang des Dosisanzeigeglieds, vorzugsweise wendelförmig, erstrecken. Die Dosisskala umfasst vorzugsweise eine Vielzahl von Dosiswerten, die aneinandergereiht angeordnet sind und die Dosisskala ergeben. Vorzugsweise handelt es sich um Zahlenwerte, welche die gewünschte Produktdosis in internationalen Einheiten (IU) oder in mg angeben.

Alternativ kann die Dosisskala ohne Steigung über den Umfang des Dosisanzeigeglieds, wie zum Beispiel des Dosisanzeigerings angeordnet sein, wobei sich die Skalenwerte dann nach einer Umdrehung des Dosisanzeigeglieds wiederholen. Bei einer Dosisskala mit Steigung, d. h. mit einer wendelförmigen Dosisskala kann das Dosisanzeigeglied, insbesondere die Dosisanzeigetrommel mit mehr als einer Umdrehung gedreht werden, ohne dass sich die Skalenwerte wiederholen, wodurch vorteilhaft die Skalenwerte größer oder mehr Skalenwerte dargestellt werden können.

Das Dosisanzeigeglied kann insbesondere in der unbetätigten Position des Betätigungsglieds, so, wie zum Beispiel mittelbar oder unmittelbar, mit dem Dosiseinstellglied gekoppelt sein, dass eine Drehung des Dosiseinstellglieds eine Drehung des Dosisanzeigeglieds bewirkt. Insbesondere können das Dosisanzeigeglied und das Dosiseinstellglied miteinander drehfest gekoppelt oder verbunden sein, zumindest wenn das Betätigungsglied in seiner unbetätigten Position ist. Beispielsweise kann eine Kupplung vorgesehen sein, welche die Bewegung betreffend, kinematisch, zwischen dem Dosiseinstellglied und dem Dosisanzeigeglied angeordnet ist, wobei die Kupplung eingekuppelt ist, wenn sich das Betätigungsglied in seiner unbetätigten Position befindet, wodurch Drehmoment von dem Dosiseinstellglied auf das Dosisanzeigeglied übertragen werden kann. Die Kupplung kann ausgekuppelt sein, wenn das Betätigungsglied in seiner betätigten Position ist, wodurch zwischen Dosisanzeigeglied und Dosiseinstellglied kein Drehmoment übertragbar ist.

Mit anderen Worten kann über das Dosiseinstellglied die einzustellende Produktdosis eingestellt werden, wobei die Drehung des Dosiseinstellglieds eine Drehung des Dosisanzeigeglieds bewirkt, wodurch die eingestellte Produktdosis anhand eines Skalenwerts von einer am Außenumfang des Dosisanzeigeglieds angeordneten Skala über die Zeigeeinrichtung ablesbar ist.

Die Zeigeeinrichtung, insbesondere das Fenster, kann von dem Gehäuse gebildet sein. In Weiterbildungen der Erfindung kann die Zeigeeinrichtung, insbesondere das Fenster, von dem Betätigungsglied, wie zum Beispiel dessen Hauptabschnitt, gebildet sein. Die Zeigeeinrichtung, insbesondere das Fenster kann somit durch Verschieben des Betätigungsabschnitts bzw. des Betätigungsglieds mitverschoben werden.

In Weiterbildungen kann das Betätigungsglied so mit dem Dosisanzeigeglied gekoppelt sein, dass das Dosisanzeigeglied die Bewegung des Betätigungsglieds mitmacht, wenn das Betätigungsglied aus der unbetätigten Position in die betätigte Position verschoben oder aus der betätigten Position in die unbetätigte Position und zurückverschoben wird. Beispielsweise kann das Dosisanzeigeglied, wie zum Beispiel die Dosisanzeigetrommel ein Außengewinde aufweisen, welches eine Steigung aufweisen kann, die der Steigung der helixförmigen Dosisskala entspricht. Das Betätigungselement, wie zum Beispiel ein Hauptabschnitt des Betätigungselements, kann zum Beispiel mittels eines Innengewindes oder mindestens eines Gewindesegments in das Außengewinde des Dosisanzeigeglieds eingreifen, wodurch das Dosisanzeigeglied an dem Betätigungselement entlang geschraubt wird, wenn das Dosisanzeigeglied relativ zu dem Betätigungselement, wie zum Beispiel zur Dosiseinstellung, gedreht wird. Wenn das Dosisanzeigeglied ein oder mehrere Dosisanzeigeringe umfasst, kann dieser oder können diese drehbar und axialfest an dem Betätigungsglied gelagert sein, insbesondere an dessen Hauptabschnitt.

Das Betätigungsglied kann in Bezug auf das Gehäuse um die Längsachse der Antriebs- und Dosiervorrichtung drehfest und entlang der Längsachse verschiebbar sein. Hierzu kann die Antriebs- und Dosiervorrichtung, insbesondere deren Gehäuse, eine Führung aufweisen, welche das Betätigungsglied in Bezug auf das Gehäuse verdrehfest und axial verschiebbar führt.

Optional kann die Antriebs- und Dosiervorrichtung oder deren Gehäuse ein Sichtfenster, wie zum Beispiel ein zusätzliches Sichtfenster, aufweisen, wobei das Betätigungsglied eine innerhalb des Gehäuses angeordnete Markierung insbesondere eine Betätigungs-Markierung aufweisen kann. Die Markierung kann eine farbliche Markierung, eine Zahl, ein Buchstabe, ein Piktogramm oder dergleichen sein. Die Markierung kann so an dem Betätigungsglied angeordnet sein, dass sie in der unbetätigten Position des Betätigungsglieds in Bezug auf das Sichtfenster so positioniert ist, dass sie durch das Sichtfenster ablesbar ist. Beispielsweise kann die Markierung in Blickrichtung von außen unter dem Sichtfenster angeordnet sein, so dass die Markierung durch das Sichtfenster sichtbar bzw. ablesbar ist. Die Markierung kann zusammen mit dem Betätigungsglied durch das Verschieben des Betätigungsglieds in die betätigte Position in Bezug auf das Sichtfenster in eine Position verschoben werden, in der sie durch das Sichtfenster nicht mehr ablesbar ist. Mit anderen Worten wird durch das Verschieben des Betätigungsglieds aus der unbetätigten Position die Markierung aus der Position unterhalb des Sichtfensters verschoben, so dass sie nicht mehr sichtbar ist. Hierdurch kann dem Benutzer eine zusätzliche optische Überwachung des Betätigungszustands bereitgestellt werden.

Alternativ oder zusätzlich zu den Markierungen an dem Betätigungsglied kann das Dosisanzeigeglied eine innerhalb des Gehäuses angeordnete Nulldosis-Markierung aufweisen. Die Nulldosis-Markierung kann eine farbliche Markierung, ein Buchstabe, eine Zahl, ein Piktogramm oder dergleichen sein. Die Nulldosis-Markierung kann insbesondere unterschiedlich von der Skala sein, insbesondere des Skalenwerts, der die Nulldosis in der Zeigeeinrichtung anzeigt, wenn zum Beispiel keine Dosis eingestellt ist oder die Dosis vollständig ausgeschüttet wurde. Insbesondere ist die Nulldosis-Markierung von dem Skalenwert Null winkelversetzt und/oder axial versetzt an dem Dosisanzeigeglied angeordnet.

Die Nulldosis-Markierung ist so an dem Dosisanzeigeglied angeordnet, dass sie, wenn die eingestellte Dosis Null ist oder die eingestellte Dosis vollständig verabreicht wurde in Bezug auf das Sichtfenster so positioniert ist, dass sie durch das Sichtfenster ablesbar ist. Bei dem Sichtfenster kann es sich um das gleiche oder um ein unterschiedliches Sichtfenster wie das Sichtfenster, in dem die Markierung des Betätigungsglieds anzeigbar ist, sein. Insbesondere wenn das Sichtfenster das gleiche Sichtfenster ist, kann als zusätzliche Bedingung, dass die Nulldosis-Markierung sichtbar ist, hinzukommen, dass das Betätigungsglied in seiner betätigten Position ist, da zum Beispiel die Betätigungsmarkierung die Nulldosis-Markierung abdecken kann, insbesondere, dass die Nulldosis-Markierung verschoben wird, wenn die Betätigungs-Markierung im Sichtfenster erscheint bzw wenn das Betätigungsglied in seiner unbetätigten Position ist. In diesem Fall würde nicht die Nulldosis-Markierung sondern die Markierung des Betätigungsglieds angezeigt werden.

Der Vorteil an einer von dem Dosiswert Null separaten Nulldosis-Markierung ist, dass der Benutzer eine zusätzliche Anzeige darüber erhält, ob die Produktausschüttung vollständig beendet wurde. Das kann auch vorteilhaft sein, wenn der Betätigungsabschnitt am Betätigungsglied so angeordnet ist, dass der Benutzer der Vorrichtung mit einem Teil seiner Hand die Zeigeeinrichtung verdecken würde, wenn er das Betätigungsglied betätigt. In Weiterbildungen der Erfindung können mehrere Nulldosis Markierungen und Sichtfenster insbesondere zwei, drei oder vier vorgesehen sein, welche über den Umfang der Antriebs-und Dosiervorrichtung insbesondere gleichmässig verteilt oder axial versenkt sind.

Das Sichtfenster, insbesondere das Sichtfenster für die Betätigungs-Markierung des Betätigungsglieds und/oder das Sichtfenster für die Nulldosis-Markierung des Dosisanzeigeglieds, kann proximal oder distal des Betätigungsabschnitts sein. Zum Beispiel kann das Sichtfenster am Dosiseinstellglied angebracht sein, zwischen dem Betätigungsabschnitt und dem Dosiseinstellglied angeordnet sein oder zwischen dem Betätigungsabschnitt und einer Schutzkappe sein. Das Gehäuse kann beispielsweise einen Durchbruch aufweisen, wobei der Betätigungsabschnitt einen Vorsprung aufweist, der von einem innerhalb des Gehäuses gebildeten Hauptabschnitt des Betätigungsglieds abragt. Der Hauptabschnitt kann zum Beispiel die Zeigeeinrichtung bilden und/oder an dem Gehäuse längsgeführt sein. Der Betätigungsabschnitt kann sich durch den Gehäusedurchbruch erstrecken, wobei das freie Ende des Betätigungsabschnitts, d. h. das Ende, welches von dem Hauptabschnitt weg weist, über den Außenumfang des Gehäuses ragt oder absteht. Dadurch kann der Benutzer den quer zur Längsrichtung von dem Hauptabschnitt abragenden Betätigungsabschnitt zum Beispiel mit einem Finger betätigen, insbesondere in die distale oder proximale Richtung drücken. In dieser Ausführung kann zum Beispiel der Hauptabschnitt einen Durchbruch insbesondere eine Zeigeeinrichtung aufweisen, an dem Gehäuse geführt werden, innerhalb des Gehäuses angeordnet sein und mit einer hierin beschriebenen Art in einem Eingriff mit dem Dosisanzeigeglied sein. Alternativ kann das Betätigungsglied so ausgestaltet sein, dass der Benutzer zum Auslösen der Injektionsvorrichtung das Betätigungsglied quer zur Längsachse hin verschiebt, insbesondere quer oder radial in das Gehäuse hineindrückt.

In Ausführungen kann das Gehäuse einen Durchbruch aufweisen, wobei der Hauptabschnitt des Betätigungsglieds außerhalb des Gehäuses angeordnet ist und zum Beispiel axialbewegbar, d.h. entlang der Längsachse verschiebbar, mit dem Gehäuse verschnappt ist. Der Betätigungsabschnitt kann ein von dem Hauptabschnitt nach außen ragender Vorsprung sein, den der Benutzer mit einem Finger betätigen kann. Der Hauptabschnitt kann auch in dieser Ausführung die Zeigeeinrichtung bilden.

Der Betätigungsabschnitt kann proximal oder distal der Zeigeeinrichtung angeordnet sein. D. h., dass der Betätigungsabschnitt zwischen der Zeigeeinrichtung und dem Dosiseinstellglied oder zwischen dem Betätigungsglied und dem distalen Ende der Antriebs- und Dosiervorrichtung angeordnet sein kann. Eine Anordnung des Betätigungsabschnitts proximal der Zeigeeinrichtung hat den Vorteil, dass die Zeigeeinrichtung beim Betätigen besser ablesbar ist und nicht von einem Teil der Hand verdeckt wird. Die Anordnung des Betätigungsabschnitts distal der Zeigeeinrichtung hat den Vorteil, dass ein längerer Gehäuseabschnitt zum Ergreifen der Antriebs- und Dosiervorrichtung bei gleichzeitiger Ausschüttung mit einem Finger zur Verfügung steht.

Der Betätigungsabschnitt kann in Umfangsrichtung oder um die Längsachse auf der gleichen Winkelposition wie die Zeigeeinrichtung angeordnet sein. Dies hat den Vorteil, dass das Betätigungsglied kompakt baut. Alternativ kann der Betätigungsabschnitt in Umfangsrichtung oder um die Längsrichtung winkelversetzt zu der Zeigeeinrichtung angeordnet sein. Dies hat den Vorteil, dass die Zeigeeinrichtung während der Produktausschüttung besser ablesbar ist, d. h. nicht durch einen Teil der Hand, welche die Antriebs- und Dosiervorrichtung umgreift, verdeckt wird.

Das Betätigungsglied kann das Gehäuse über seinen Umfang teilweise, wie zum Beispiel über den überwiegenden Teil, wie zum Beispiel ringsegmentförmig, oder vollständig, wie zum Beispiel ringförmig, umgeben. Diese Ausführungen haben den Vorteil, dass der Betätigungsabschnitt vom Benutzer in einer Vielzahl von Winkelpositionen, den die Hand in Bezug auf das Gehäuse einnimmt, betätigt werden kann. Somit wird eine einfachere Bedienung bewirkt.

Das Dosisanzeigeglied kann zwischen einer Maximaldosisposition (Maximaldosisanschlag) und einer Nulldosisposition (Nulldosisanschlag) hin und her schraubbar sein. In der Nulldosisposition kann zum Beispiel die Dosis oder die Ziffer "Null" in der Anzeigeeinrichtung ablesbar sein. In der Maximaldosisposition kann vorteilhaft die maximal mit der Antriebs- und Dosiervorrichtung ausschüttbare Produktdosis ablesbar sein.

In der Nulldosisposition kann das Dosisanzeigeglied gegen die Drehung in eine, z. B. zweite, Drehrichtung gesperrt sein, nämlich in die Drehrichtung, die bewirken würde, dass eine Dosis kleiner als Null eingestellt wird. In der Nulldosisposition kann das Dosisanzeigeglied beispielsweise nur in die, z. B. erste, Drehrichtung bewegt werden, die eine Erhöhung der Dosis bewirkt. In der Maximaldosisposition kann das Dosisanzeigeglied beispielsweise gegen die Drehung in eine, z. B. erste, Drehrichtung, nämlich in die Drehrichtung, die eine Einstellung der Dosis über die maximal einstellbare Dosis hinaus bewirken würde, blockiert sein. Das Dosisanzeigeglied kann in der Maximaldosisposition beispielsweise nur in die, z.B. zweite, Drehrichtung gedreht werden, die eine Verringerung der Produktdosis bewirkt.

Das Dosisanzeigeglied kann beispielsweise einen Anschlag, beispielsweise einen Nulldosisanschlag, aufweisen, der in der Nulldosisposition an einen Gegenanschlag, beispielsweise einen Nulldosisgegenanschlag, der Antriebs- und Dosiervorrichtung anschlägt und somit die Drehung in die zweite Drehrichtung verhindert. Der gleiche oder ein zusätzlicher Anschlag, beispielsweise ein Maximaldosisgegenanschlag, des Dosisanzeigeglieds kann die Drehung des Dosisanzeigeglieds über die Maximaldosis hinaus bzw. in die erste Drehrichtung verhindern, indem der Anschlag an einen Maximaldosisgegenanschlag der Antriebs- und Dosiervorrichtung anschlägt. Das Dosisanzeigeglied kann demnach einen Nulldosisanschlag für den Nulldosisgegenanschlag und einen Maximaldosisanschlag für den Maximaldosisgegenanschlag aufweisen. Vorzugsweise wirken der Anschlag oder die Anschläge in Umfangsrichtung und/oder in Axialrichtung. Zusätzlich kann die Antriebs- und Dosiervorrichtung eine Überlastkupplung aufweisen. Dies hat den Vorteil, dass bei einer Überdrehung des Dosiermechanismus, bei der sich die Vorrichtung z.B. in einem Stoppanschlag befindet, ein Schaden an der Vorrichtung verhindert wird

Die Antriebs- und Dosiervorrichtung kann ein im Gehäuse aufgenommenes Abtriebsglied, wie zum Beispiel eine Gewinde- oder Kolbenstange umfassen, deren distales Ende unmittelbar oder mittelbar, wie zum Beispiel über einen am distalen Ende der Gewinde oder Kolbenstange angebrachten tellerförmigen Flansch, auf einen in dem Produktbehälter verschiebbar aufgenommenen Kolben wirken kann, um diesen für die Produktausschüttung zu verschieben. Das Abtriebsglied kann zum Beispiel für die Produktausschüttung eine in die distale Richtung gerichtete Schraubbewegung insbesondere um die Längsachse ausführen. Hierzu kann die Kolben- oder Gewindestange an ihrem Umfang ein Außengewinde aufweisen, welches in ein Gewinde des Gehäuses oder eines gehäusefesten Elements eingreift, wodurch eine Drehung der Kolben- oder Gewindestange eine Schraubbewegung der Kolben- oder Gewindestange bewirkt.

Alternativ kann die Kolben- oder Gewindestange eine Längsführung, wie zum Beispiel eine Längsnut oder mindestens eine abgeflachte Flanke aufweisen, in die ein Eingriffselement des Gehäuses oder des gehäusefesten Elements eingreift, wodurch die Kolben- oder Gewindestange in Bezug auf das Gehäuse verdrehgesichert aber axial verschiebbar ist. Hierdurch führt das Abtriebsglied für die Produktausschüttung eine reine Axialbewegung (ohne Drehbewegung) aus.

Die Antriebs- und Dosiervorrichtung kann beispielsweise eine Antriebsfeder aufweisen, insbesondere eine vorspannbare oder vorgespannte Antriebsfeder, welche die für die Produktausschüttung erforderliche Energie speichern und an ein Antriebsglied und somit auch an das Abtriebsglied abgegeben kann. Die Antriebsfeder kann in einer Variante so mit dem Dosiseinstellglied gekoppelt sein, dass die Antriebsfeder durch Drehen des Dosiseinstellglieds, insbesondere in eine Drehrichtung, welche eine Erhöhung der Dosis bewirkt (erste Drehrichtung), gespannt wird. Optional kann die Antriebsfeder durch Drehen des Dosiseinstellglieds in die Drehrichtung, welche eine Dosisverringerung bewirkt (zweite Drehrichtung), entspannt werden oder nicht entspannt werden. Wenn die Feder nicht entspannt wird, kann zwischen dem Dosiseinstellglied und der Feder eine Ratsche angeordnet sein, welche bewirkt, dass die Feder durch Drehen des Dosiseinstellglieds nur gespannt aber nicht mehr entspannt werden kann.

In einer alternativen Variante kann die Antriebsfeder eine vorgespannte Antriebsfeder sein, die beispielsweise so stark vorgespannt ist, dass die in ihr gespeicherte Federenergie ausreicht, um das Produkt aus einem vollständig gefüllten Produktbehälter in einem oder mehreren Ausschüttungen vollständig auszuschütten. Hierzu kann die Antriebsfeder so angeordnet sein, dass eine Drehung des Dosiseinstellglieds kein Spannen oder Entspannen der Feder bewirkt. D. h., dass bei der Drehung des Dosiseinstellglieds in die erste Drehrichtung, welche eine Dosiserhöhung bewirkt, kein Spannen der Feder stattfindet (und auch kein Entspannen) und durch Drehen des Dosiseinstellglieds in die zweite Drehrichtung, welche eine Dosisverringerung bewirkt, kein Entspannen (und auch kein Spannen) der Feder bewirkt wird.

Die Antriebs- und Dosiervorrichtung kann ein Antriebsglied umfassen, welches relativ zu dem Gehäuse und vorzugsweise um die Längsachse drehbar und während der Produktausschüttung oder bei betätigtem Betätigungsglied so mit dem Abtriebsglied gekoppelt ist, dass eine Drehung des Antriebsglieds bewirkt, dass das Abtriebsglied relativ zu dem Gehäuse in die distale Richtung bewegt wird. Ein Ende der Antriebsfeder, die auch als Dreh- oder Torsionsfeder bezeichnet werden kann, kann an dem Antriebsglied befestigt sein oder sich an dem Antriebsglied abstützen. Die mindestens eine Antriebsfeder, welche als Ausschüttfeder dient, kann eine Wendel- oder Schraubenfeder sein, die als Dreh- oder Torsionsfeder wirkt. Besonders bevorzugt kann die Antriebsfeder eine spiralförmig aus einem bandförmigen Material, insbesondere Metall gewickelte Feder sein, die zum Beispiel als Spiral- oder Uhrenfeder bezeichnet werden kann. Eine rotatorisch vorgespannte Feder versucht die Teile, an denen sie sich abstützt, relativ zueinander zu verdrehen. Das andere Ende der Feder kann sich mittelbar oder unmittelbar an dem Gehäuse, einem gehäusefesten Element, dem Dosiseinstellglied oder einem zumindest in eine Richtung mit dem Dosiseinstellglied drehfesten Element, wie zum Beispiel einem Federgehäuse, abstützen.

Beispielsweise kann die Bewegung betreffend, d. h. kinematisch zwischen dem Antriebsglied und dem Abtriebsglied ein Rotationsglied angeordnet sein. Das bevorzugt hülsenförmige Rotationsglied kann das Abtriebsglied umgeben. Das Rotationsglied ist vorzugsweise axialfest und drehbar mit dem Gehäuse oder einem gehäusefesten Element verbunden. Vorzugsweise greifen das Gehäuse oder ein gehäusefestes Element und das Rotationsglied so ineinander, dass das Rotationsglied relativ zu dem Gehäuse drehbar und axialfest, d.h. entlang der Längsachse unverschiebbar ist.

In einer ersten Variante kann das Rotationsglied in einem Gewindeeingriff mit einem Gewinde des Abtriebsglieds sein. Beispielsweise kann das Abtriebsglied ein Innengewinde und das Rotationsglied ein Außengewinde aufweisen, die ineinandergreifen. Bevorzugt kann das Rotationsglied ein Innengewinde und das Abtriebsglied, insbesondere dessen Gewindestange, ein Außengewinde aufweisen, die ineinandergreifen.

Das Abtriebsglied, wie z. B. dessen Gewindestange, kann mit einer gehäusefesten Führung der Antriebs- und Dosiervorrichtung in einem Eingriff sein. Zum Beispiel kann die gehäusefeste Führung eine Nocke oder ein unrunder Querschnitt sein, der in eine Nut oder einen unrunden Querschnitt, wie zum Beispiel mindestens eine abgeflachte Flanke, des Abtriebsglieds eingreift. Zum Beispiel kann sich die Nut oder der unrunde Querschnitt parallel zur Längsachse des länglichen Abtriebsglieds erstrecken. Die gehäusefeste Führung kann von dem Gehäuse selbst oder einem dreh- oder axialfest mit dem Gehäuse verbundenen Teil gebildet werden. Dieses Teil kann permanent oder zumindest während der Produktausschüttung drehund axialfest mit dem Gehäuse verbunden sein. Die Nut oder der unrunde Querschnitt des Abtriebsglieds kann sich alternativ zu der Ausführung in der sie bzw. er sich parallel zur Längsachse erstreckt, helix- oder wendelförmig um die Längsachse erstrecken, jedoch mit einer anderen Steigung als das Gewinde. Diese Varianten bewirken, dass eine Drehung des Rotationsglieds eine Bewegung des Abtriebsglieds in die distale Richtung bewirkt, insbesondere wenn das Rotationsglied in die zweite Drehrichtung relativ zu dem Gehäuse gedreht wird. Hierbei kann sich das Abtriebsglied entweder in die distale Richtung schrauben, insbesondere wenn die Nut oder der unrunde Querschnitt des Abtriebsglieds helixförmig ist, oder linear verschieben, insbesondere wenn die Nut oder der unrunde Querschnitt sich parallel zur Längsachse erstreckt.

In einer zweiten Variante kann ein gehäusefestes Innengewinde in einem Gewindeeingriff mit einem Außengewinde des Abtriebsglieds, insbesondere dessen Gewindestange, sein. Das Abtriebsglied, insbesondere die Gewindestange, und das Rotationsglied können so ineinandergreifen, dass das Abtriebsglied relativ zum Rotationsglied drehfest und entlang der Längsachse verschiebbar ist. Eine Drehung des Rotationsglieds bewirkt eine Schraubbewegung des Abtriebsglieds in die distale Richtung. Das Abtriebsglied kann einen unrunden Querschnitt oder eine Nut aufweisen, der oder die sich entlang oder parallel der Längsachse erstreckt und in einem Eingriff mit einem unrunden Querschnitt oder einer Abragung des Rotationsglieds ist.

Allgemein bevorzugt bewirkt eine Drehung des Antriebsglieds in die zweite Drehrichtung während der Produktausschüttung oder wenn das Betätigungsglied betätigt ist, relativ zu dem Gehäuse und/oder dem Dosiseinstellglied, dass das Abtriebsglied relativ zu dem Gehäuse in die distale Richtung bewegt wird. Insbesondere bewirkt eine Drehung des Antriebsglieds relativ zu dem Gehäuse und/oder dem Dosiseinstellglied, dass das kinematisch zwischen dem Antriebsglied und dem Abtriebsglied angeordnete Rotationsglied mit dem Antriebsglied mitgedreht wird, d. h. relativ zu dem Gehäuse und/oder dem Dosiseinstellglied gedreht wird. Die Drehung des Rotationsglieds in die zweite Drehrichtung bewirkt eine Bewegung des Abtriebsglieds in die distale Richtung. Das Abtriebsglied wird insbesondere nur während der Produktausschüttung relativ zu dem Gehäuse in die distale Richtung bewegt.

Die Antriebs- und Dosiervorrichtung kann beispielsweise eine erste Kupplung aufweisen, welche zwischen, insbesondere kinematisch zwischen dem Antriebsglied und dem Abtriebsglied angeordnet ist. Insbesondere kann die erste Kupplung zwischen dem Antriebsglied und dem Rotationsglied angeordnet sein. Die erste Kupplung ist während der Einstellung der Produktdosis oder bei unbetätigtem Betätigungsglied ausgekuppelt, wodurch das Antriebsglied relativ zu dem Abtriebsglied verdrehbar ist. Während der Produktausschüttung oder bei betätigtem Betätigungsglied ist die erste Kupplung eingekuppelt. Hierdurch wird insbesondere erreicht, dass das Rotationsglied und das Antriebsglied verdrehfest verbunden sind. Das Rotationsglied kann somit mit dem Antriebsglied beispielsweise in die zweite Drehrichtung mitgedreht werden.

Die erste Kupplung kann eine erste Kupplungsstruktur, welche verdrehgesichert mit dem Antriebsglied verbunden ist, insbesondere von dem Antriebsglied gebildet wird, und eine zweite Kupplungsstruktur, welche verdrehfest mit dem Rotationsglied verbunden ist, insbesondere von dem Rotationsglied gebildet wird, umfassen. Die erste Kupplungsstruktur kann eine Verzahnung sein oder aufweisen, wobei die zweite Kupplungsstruktur eine Verzahnung sein oder aufweisen kann. Die Verzahnungen der ersten Kupplungsstruktur und der zweiten Kupplungsstruktur können formschlüssig ineinandergreifen, wenn die erste Kupplung eingekuppelt ist. Zum Beispiel kann die erste Kupplungsstruktur eine Innenverzahnung sein, wobei die zweite Kupplungsstruktur eine Außenverzahnung sein kann. Alternativ kann die erste Kupplungsstruktur eine Außenverzahnung sein, wobei die zweite Kupplungsstruktur eine Innenverzahnung sein kann.

Das Betätigungsglied kann - mittelbar oder unmittelbar - mit der ersten Kupplungsstruktur beispielsweise axialfest verbunden sein, so dass sich die erste Kupplungsstruktur mit dem Betätigungsglied mitverschieben lässt, wodurch mittels Verschieben des Betätigungsglieds aus der unbetätigten Position in die betätigte Position die erste Kupplung eingekuppelt wird, d. h. dass die erste Kupplungsstruktur in den Eingriff mit der zweiten Kupplungsstruktur verschoben wird.

Die Antriebs- und Dosiervorrichtung kann beispielsweise eine zweite Kupplung aufweisen, welche insbesondere dazu dient, dass sich die Antriebsfeder nicht unkontrolliert entspannen kann. Die zweite Kupplung kann zwischen, insbesondere kinematisch zwischen dem Dosiseinstellglied und dem Antriebsglied angeordnet sein. Die zweite Kupplung ist während der Einstellung der Produktdosis oder bei unbetätigtem Betätigungsglied eingekuppelt, wodurch das Dosiseinstellglied und das Antriebsglied in Bezug zueinander verdrehgesichert sind. Die zweite Kupplung kann eine dritte Kupplungsstruktur, die verdrehfest mit dem Dosiseinstellglied verbunden ist, insbesondere von dem Dosiseinstellglied gebildet wird, und eine vierte Kupplungsstruktur, die verdrehfest mit dem Antriebsglied verbunden ist, insbesondere von dem Antriebsglied gebildet wird, aufweisen.

In einer anderen Ausführung kann die zweite Kupplung zwischen, insbesondere kinematisch zwischen dem Antriebsglied und dem Gehäuse, einem gehäusefesten Element oder einem Element, welches formschlüssig in das Gehäuse oder das gehäusefeste Element eingreift und dessen formschlüssiger Eingriff durch seine Drehung relativ zu dem Gehäuse lösbar ist, wie zum Beispiel einem Kupplungsglied, angeordnet sein. Die zweite Kupplung ist während der Einstellung der Produktdosis oder bei unbetätigtem Betätigungsglied eingekuppelt, wodurch das Antriebsglied relativ zu dem Kupplungsglied nicht drehbar ist. Das Kupplungsglied kann eine Eingriffsstruktur und das Gehäuse kann eine Eingriffsgegenstruktur aufweisen, welche formschlüssig ineinandergreifen, insbesondere von einem Elastizitätsmittel, z.B. einer Feder, axial in den Eingriff gedrückt werden, wobei die Eingriffsstruktur und die Eingriffsgegenstruktur und ggf. das Elastizitätsmittel so aufeinander abgestimmt sind, dass für die Drehung des Kupplungsglieds in die erste Drehrichtung ein auf das Kupplungsglied in die erste Drehrichtung wirkendes erstes Grenzmoment überschritten werden muss und für die Drehung des Kupplungsglieds in die zweite Drehrichtung ein auf das Kupplungsglied in die zweite Drehrichtung wirkendes zweites Grenzmoment überschritten werden muss. Das erste Grenzmoment kann geringer sein als das zweite Grenzmoment. Die Abstimmung ist so, dass das zweite Grenzmoment größer ist als das Drehmoment, mit dem die Antriebsfeder mittels der Antriebs- und Dosiervorrichtung maximal gespannt werden kann, d.h. das Drehmoment, welches die Antriebsfeder bereitstellt, wenn der Maximaldosisanschlag und der Maximaldosisgegenanschlag aneinander anliegen. Dadurch wird bewirkt, dass das von der Antriebsfeder bereitgestellte Drehmoment alleine nicht ausreicht, das Kupplungsglied relativ zu dem Gehäuse in die zweite Drehrichtung zu verdrehen, sondern dass eine Verdrehung in die zweite Drehrichtung nur mit einem zusätzlichen, in die zweite Drehrichtung wirkenden Drehmoment, welches zum Beispiel vom Benutzer auf das Dosiseinstellglied aufgebracht wird, möglich ist. Hierdurch wird ein unkontrolliertes Entspannen der Antriebsfeder verhindert. Die zweite Kupplung kann eine dritte Kupplungsstruktur, die verdrehfest mit dem Kupplungsglied verbunden ist, insbesondere von dem Kupplungsglied gebildet wird, und eine vierte Kupplungsstruktur, die verdrehfest mit dem Antriebsglied verbunden ist, insbesondere von dem Antriebsglied gebildet wird, aufweisen.

Während der Produktausschüttung oder bei betätigtem Betätigungsglied ist die zweite Kupplung ausgekuppelt, wodurch das Antriebsglied mittels der vorgespannten Antriebsfeder relativ zu dem Dosiseinstellglied, insbesondere in die zweite Drehrichtung, drehbar ist.

Die dritte Kupplungsstruktur kann eine Verzahnung sein oder aufweisen, wobei die vierte Kupplungsstruktur eine Verzahnung sein oder aufweisen kann. Die Verzahnungen der dritten Kupplungsstruktur und der vierten Kupplungsstruktur können formschlüssig ineinandergreifen, wenn die zweite Kupplung eingekuppelt ist. Die dritte Kupplungsstruktur kann eine Innenverzahnung sein, wobei die vierte Kupplungsstruktur eine Außenverzahnung sein kann. Alternativ kann die dritte Kupplungsstruktur eine Außenverzahnung sein, wobei die vierte Kupplungsstruktur eine Innenverzahnung sein kann.

Die vierte Kupplungsstruktur kann mit dem Betätigungsglied axialfest verbunden sein, wodurch die vierte Kupplungsstruktur die Bewegung des Betätigungsglieds mitmacht. Insbesondere kann die vierte Kupplungsstruktur mittels verschieben des Betätigungsglieds aus der unbetätigten Position in die betätigte Position aus dem Eingriff mit der dritten Kupplungsstruktur bewegt werden, wodurch die zweite Kupplung ausgekuppelt wird.

Die erste und zweite Kupplung können beispielsweise so aufeinander abgestimmt sein, dass das Betätigungsglied zwischen der unbetätigten Position und der betätigten Position eine, zum Beispiel erste, Zwischenposition einnehmen kann, in welche die erste Kupplung eingekuppelt ist und die zweite Kupplung eingekuppelt ist. Dies bewirkt vorteilhaft, dass die Drehung des Antriebsglieds durch die Antriebsfeder erst dann freigegeben wird, wenn sichergestellt ist, dass das Rotationsglied drehfest mit dem Antriebsglied gekoppelt ist. Hierdurch wird vorteilhaft eine Fehlfunktion der Vorrichtung vermieden, die auftreten könnte, wenn die erste Kupplung noch nicht eingekuppelt ist und die zweite Kupplung bereits ausgekuppelt ist.

Beispielsweise kann zwischen dem Rotationsglied und dem Gehäuse oder einem gehäusefesten Element eine unidirektionale Kupplung, wie zum Beispiel eine Ratsche, angeordnet sein, welche die Drehung des Rotationsglieds relativ zu dem Gehäuse in z.B. die zweite Drehrichtung zulässt und die andere, wie z.B. die erste Drehrichtung blockiert. Vorzugsweise lässt die unidirektionale Kupplung die Drehung des Rotationsglieds relativ zu dem Gehäuse in die Richtung zu, welche die Bewegung des Abtriebsglieds in die distale Richtung bewirkt.

Optional kann die Antriebs- und Dosiervorrichtung eine dritte Kupplung aufweisen, welche zwischen, insbesondere kinematisch zwischen dem Dosiseinstellglied und dem Gehäuse angeordnet sein kann. Die dritte Kupplung ist während der Einstellung der Produktdosis oder bei unbetätigtem Betätigungsglied ausgekuppelt, wodurch das Dosiseinstellglied relativ zu dem Gehäuse verdrehbar ist. Während der Produktausschüttung oder bei betätigtem Betätigungsglied ist die dritte Kupplung eingekuppelt, wodurch das Dosiseinstellglied relativ zu dem Gehäuse drehfest ist. Allgemein ist bevorzugt, dass das Dosiseinstellglied während der Produktausschüttung relativ zu dem Gehäuse verdrehfest ist. Dies bewirkt einerseits vorteilhaft, dass während der Produktausschüttung eine Dosiseinstellung nicht möglich ist und anderseits, dass sich die Antriebsfeder mittelbar mit einem Abschnitt an dem Gehäuse abstützen kann.

Die dritte Kupplung kann zwischen dem Dosiseinstellglied oder dem Federgehäuse und dem Gehäuse gebildet sein, wobei bevorzugt ist, dass das Federgehäuse und das Dosiseinstellglied verdrehfest, insbesondere permanent verdrehfest oder zumindest während der Einstellung der Produktdosis und während der Produktausschüttung verdrehfest verbunden sind.

Die dritte Kupplung kann eine fünfte Kupplungsstruktur, die verdrehfest mit dem Gehäuse verbunden ist, insbesondere von dem Gehäuse gebildet wird, und eine sechste Kupplungsstruktur, die verdrehfest mit dem Dosiseinstellglied verbunden ist, insbesondere von dem Dosiseinstellglied oder dem mit dem Dosiseinstellglied drehfest verbundenen Federgehäuse gebildet wird, aufweisen. Die fünfte Kupplungsstruktur kann eine Verzahnung sein oder aufweisen, wobei die sechste Kupplungsstruktur eine Verzahnung sein oder aufweisen kann. Die Verzahnungen der fünften Kupplungsstruktur und der sechsten Kupplungsstruktur können bei eingekuppelter dritter Kupplung formschlüssig ineinandergreifen. Die fünfte Kupplungsstruktur kann eine Außenverzahnung sein, wobei die sechste Kupplungsstruktur eine Innenverzahnung sein kann. Alternativ kann die fünfte Kupplungsstruktur eine Innenverzahnung sein, wobei die sechste Kupplungsstruktur eine Außenverzahnung sein kann. Die sechste Kupplungsstruktur kann axialfest mit dem Betätigungsglied verbunden oder gekoppelt sein, wodurch die sechste Kupplungsstruktur die Bewegung des Betätigungsglieds entlang der Längsachse mitmacht. Insbesondere kann die sechste Kupplungsstruktur mittels Verschieben des Betätigungsglieds aus seiner unbetätigten Position in die betätigte Position in einen Eingriff mit der fünften Kupplungsstruktur verschoben werden, insbesondere unter Verschiebung des Federgehäuses relativ zu dem Dosiseinstellglied, wodurch die sechste Kupplungsstruktur und die fünfte Kupplungsstruktur um die Längsachse verdrehgesichert ineinandergreifen.

Das Antriebsglied kann vorzugsweise verdrehfest und axial verschiebbar mit dem Dosisanzeigeglied verbunden sein. Insbesondere können das Dosisanzeigeglied und das Antriebsglied verdrehfest und axial verschiebbar ineinandergreifen oder über ein z.B. hülsenförmiges Zwischenglied verbunden sein, wobei das Zwischenglied und das Dosisanzeigeglied drehfest und axial verschiebbar ineinandergreifen und wobei das Zwischenglied und das Antriebsglied drehfest und axial verschiebbar ineinandergreifen. An dem Zwischenglied kann z.B. die Antriebsfeder mit einem Ende befestigt sein, wobei das andere Ende der Antriebsfeder an dem Gehäuse oder einem gehäusefestem Element befestigt sein kann.

Ferner kann die Antriebs- und Dosiervorrichtung eine Letztedosiseinrichtung aufweisen, die verhindert, dass eine Dosis eingestellt werden kann, welche die in dem Produktbehälter enthaltene Produktmenge übersteigt. Die Einrichtung kommt nur dann zu tragen, wenn die in dem Produktbehälter enthaltene oder ausschüttbare Produktmenge kleiner als die mit der Antriebs- und Dosiervorrichtung einstellbare Dosis ist. Die Letztedosiseinrichtung kann vorzugsweise ein Stoppelement und einen Stoppgegenanschlag aufweisen, insbesondere kann das Stoppelement an dem Stoppgegenanschlag anschlagen wenn die in dem Produktbehälter enthaltene oder ausschüttbare Produktmenge kleiner ist, als die mit dem Antriebs- und Dosiervorrichtung einstellbare Dosis. In dieser Situation kann der Benutzer das Dosiseinstellglied nicht mehr drehen um eine Dosis einzustellen, welche grösser ist als die im Produktbehälter enthaltene oder ausschüttbare Restmenge und das Drehmoment wird vom Dosiseinstellglied über die Antriebseinrichtung an das Stoppelement- und den Stoppgegenanschlag übertragen, wobei der Stoppgegenanschlag drehfest mit dem Gehäuse verbindbar ist.

Wie bereits oben erwähnt kann die Antriebs- und Dosiervorrichtung eine Überlast- oder Sicherheitskupplung aufweisen um bei einer Überdrehung des Dosierglieds, bei der sich die Vorrichtung z.B. in einem Stoppanschlag (Nulldosisanschlag, Maximaldosisanschlag oder Letztedosisanschlag) befindet, einen Schaden an der Vorrichtung zu verhindern. Die Überlastkupplung kann bevorzugt zwischen dem Dosiseinstellglied und der Antriebseinrichtung angebracht sein. Die Überlastkupplung kann eine Ratsche oder eine federnde, RutschKupplung oder ein rastender Mechanismus sein, welcher eine Kupplung bei Überlast trennt. Falls der Benutzer das Dosiseinstellglied weiter dreht, obwohl sich die Antriebs- und Dosiereinrichtung in einem der Stoppanschläge (Nulldosisanschlag, Maximaldosisanschlag oder Letztedosisanschlag) befindet und das übertragene Drehmoment eine zulässige Grenze überschreitet, wird die Überlastkupplung aktiviert und das Antriebs- und Dosierglied voneinander trennen, bevor Drehmoment belastete Elemente beschädigt werden. Die Erfindung wurde anhand mehrerer bevorzugter Ausführungen beschrieben. Im Folgenden werden besonders bevorzugte Ausführungen der Erfindung anhand von Figuren beschrieben. Die dabei offenbarten Merkmale bilden die Erfindung einzeln und in jeglicher Merkmalskombination vorteilhaft weiter. Es zeigen:
- Figur 1: eine perspektivische Ansicht einer ersten Ausführungsform einer Injektionsvorrichtung,
- Figur 2: eine Explosionsdarstellung der Einzelteile der Vorrichtung aus Figur 1,
- Figuren 3a-3d: verschiedene Ansichten der Vorrichtung aus Figur 1 in einem Ausgangszustand,
- Figuren 4a-4d: verschiedene Ansichten der Vorrichtung aus Figur 1 in einem maximal aufdosierten Zustand und mit einem unbetätigten Betätigungsglied,
- Figuren 5a-5d: verschiedene Ansichten der Vorrichtung aus Figur 1 in einem maximal aufdosierten Zustand und mit einem betätigen Betätigungsglied,
- Figuren 6a-6d: verschiedene Ansichten der Vorrichtung aus Figur 1 in einem Zustand, in dem der Produktbehälter der Vorrichtung vollständig entleert und das Betätigungsglied unbetätigt ist,
- Figur 7: eine Explosionsdarstellung der Einzelteile einer zweiten Ausführungsform einer Injektionsvorrichtung,
- Figuren 8a-8d: verschiedene Ansichten der Ausführungsform aus Figur 7 in einem Ausgangszustand,
- Figuren 9a, 9b: verschiedene Ansichten der Ausführungsform aus Figur 7 in einem maximal aufdosierten Zustand und mit einem unbetätigten Betätigungsglied,
- Figuren 10a, 10b: verschiedene Ansichten der Ausfiihrungsfonn aus Figur 7 in einem maximal aufdosierten Zustand und mit einem betätigten Betätigungsglied,
- Figuren 11a, 11b: verschiedene Ansichten der Ausführungsform aus Figur 7 in einem Zustand, in dem der Produktbehälter der Vorrichtung vollständig entleert und das Betätigungsglied unbetätigt ist,
- Figuren 12a, 12b: zueinander um 90° um die Längsachse gedrehte Ansichten einer Injektionsvorrichtung nach einer dritten Ausführungsform,
- Figur 12c: eine perspektivische Ansicht einer dritten Ausführungsform einer Injektionsvorrichtung,
- Figuren 13a, 13b: zueinander um 90° um die Längsachse gedrehte Ansichten einer Injektionsvorrichtung nach einer vierten Ausführungsform,
- Figur 13c: eine perspektivische Ansicht einer vierten Ausführungsform einer Injektionsvorrichtung,
- Figuren 14a, 14b: zueinander um 90° um die Längsachse gedrehte Ansichten einer Injektionsvorrichtung nach einer fünften Ausführungsform,
- Figur 14c: eine perspektivische Ansicht einer fünften Ausführungsform einer Injektionsvorrichtung,
- Figuren 15a, 15b: zueinander um 90° um die Längsachse gedrehte Ansichten einer Injektionsvorrichtung nach einer sechsten Ausführungsform.
- Figur 15c: eine perspektivische Ansicht einer sechsten Ausführungsform einer Injektionsvorrichtung,

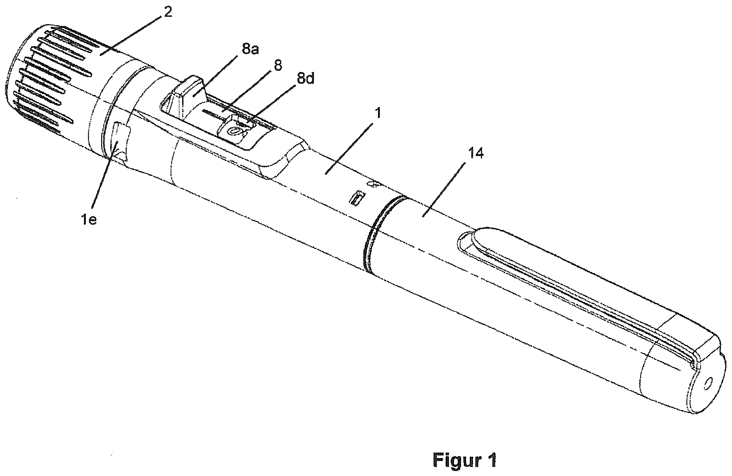

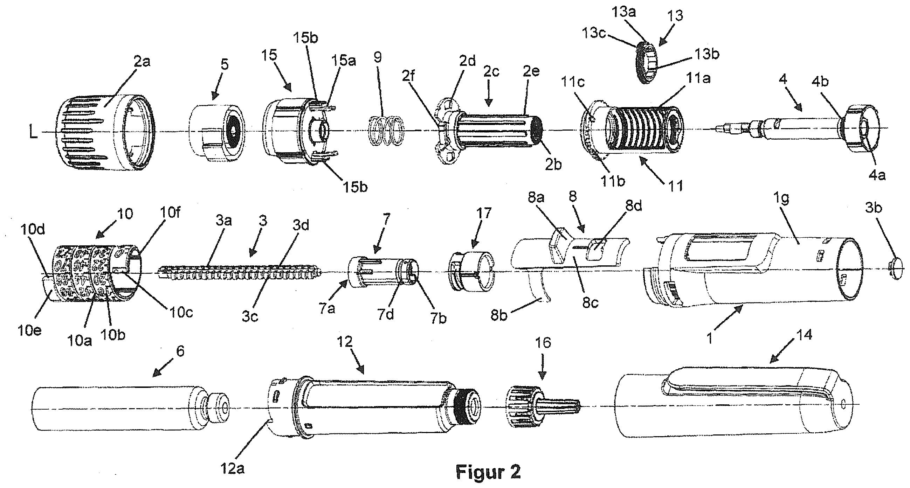

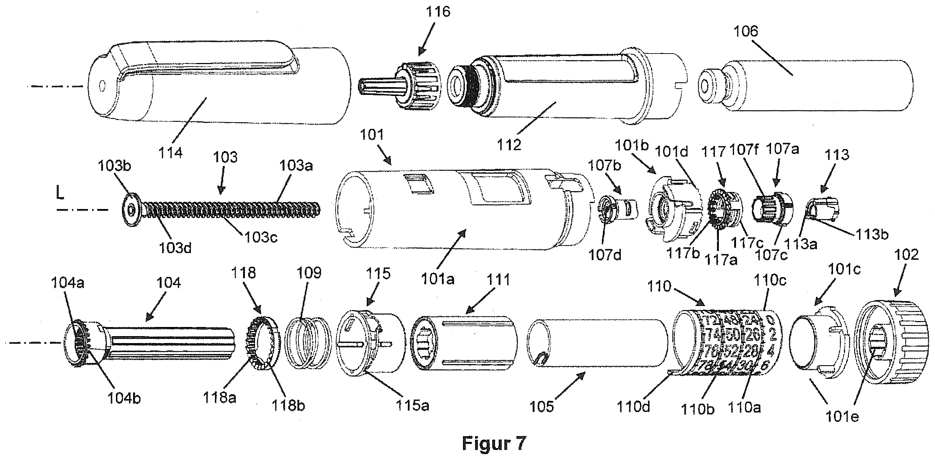

Die in den Figuren 1 bis 6d gezeigte erste Ausführungsform und die in den Figuren 7 bis 11b gezeigte zweite Ausführungsform überlappen sich nur teilweise in ihrer Funktionsweise. Die in den ersten bis sechsten Ausführungsformen gezeigten Betätigungsglieder 8, 108 können bei jeder aus erster und zweiter Ausführungsform Anwendung finden, d. h., dass jede aus erster und zweiter Ausführungsform mit einem beliebigen der hierin beschriebenen Betätigungsglieder 8, 108 modifizierbar ist. Die Antriebs- und Dosiervorrichtung bildet eine Injektionsvorrichtung oder ist zumindest ein Teil einer solchen Injektionsvorrichtung.

Die Antriebs- und Dosiervorrichtung der ersten Ausführungsform aus den Figuren 1 bis 6d weist ein hülsenförmiges Gehäuse 1 auf, welches eine Außenhülse 1g und eine damit verbundene und konzentrisch dazu angeordnete Innenhülse 1h aufweist. Die Innenhülse 1h ist mit der Außenhülse 1g dreh- und axialfest verbunden, insbesondere verschnappt. Alterativ könnte die Außenhülse 1g mit der Innenhülse 1h einteilig ausgebildet sein. Die Innenhülse 1h und die Außenhülse 1g sind über einen von der Innenhülse 1h gebildeten ringförmigen Steg fest verbunden. Das Gehäuse 1, insbesondere die Innenhülse 1h weist ein Innengewinde 1a auf, welches in ein Außengewinde 3c einer Gewindestange 3a eines Abtriebsglieds 3 eingreift, so dass die Gewindestange 3a und somit das Abtriebsglied 3 relativ zu dem Gehäuse 1 und entlang der Längsachse L in die distale Richtung geschraubt werden kann. Das Abtriebsglied 3 weist die Gewindestange 3a und einen tellerförmigen Flansch 3b auf, der frei drehbar am distalen Ende der Gewindestange 3a befestigt, insbesondere damit verschnappt ist. Die Gewindestange 3a weist mindestens eine Führungsnut 3d auf, welche das Außengewinde 3c überlagert und sich parallel zur Längsachse L erstreckt. Ein hülsenförmiges Rotationsglied 7 weist an seinem Innenumfang mindestens ein Eingriffsglied 7b auf, welches in die Führungsnut 3d eingreift, wodurch das Rotationsglied 7 und das Abtriebsglied 3 relativ zueinander verdrehfest und axial verschiebbar sind. Das Rotationsglied 7 weist an seinem Außenumfang eine ringförmige Nut 7d auf, in welche eine am Innenumfang des Gehäuses 1, insbesondere der Innenhülse 1h gebildete Abragung 1b eingreift, wodurch das Rotationsglied 7 relativ zu dem Gehäuse 1 verdrehbar und axialfest ist. Eine Drehung des Rotationsglieds 7 bewirkt eine Drehung des Abtriebsglieds 3, wodurch das Abtriebsglied 3 aufgrund des Gewindeeingriffs mit dem Gehäuse 1 entlang der Längsachse L bewegbar ist. Beispielsweise wird das Abtriebsglied 3 in die distale Richtung bewegt, wenn das Rotationsglied 7 relativ zu dem Gehäuse 1 in eine zweite Drehrichtung um die Längsachse L gedreht wird. Kinematisch zwischen dem Gehäuse 1, insbesondere der Innenhülse 1h, und dem Rotationsglied 7 ist eine unidirektionale Kupplung 17 angeordnet, welche eine Drehung des Rotationsglieds 7 in die zweite Drehrichtung erlaubt und in die erste Drehrichtung verhindert. Das Rotationsglied 7 kann sich daher nicht in die erste Drehrichtung drehen, wie zum Beispiel aufgrund von Erschütterungen oder dergleichen, wodurch bewirkt werden würde, dass sich das Abtriebsglied 3 in die proximale Richtung bewegt. Die unidirektionale Kupplung 17 kann außerdem so ausgestaltet sein, dass das Rotationsglied für die Drehung in die zweite Drehrichtung bevorzugt ein gewisses, aber kleines Drehmoment überwinden muss, wodurch verhindert wird, dass die Vibrationen eine Drehung des Rotationsglieds 7 in die zweite Drehrichtung bewirken. Die unidirektionale Kupplung 17 kann außerdem als Signalerzeugungseinrichtung ausgestaltet sein, welche bei der Drehung des Rotationsglieds 7 in die zweite Drehrichtung ein akustisches und/oder taktiles Signal (Klicks) erzeugt und dadurch die Produktausschüttung signalisiert. Die unidirektionale Kupptung 17 ist in dem gezeigten Beispiel hülsenförmig und umgibt einen Teil der Innenhülse 1h und einen Teil des Rotationsglieds 7. Die Innenhülse 1h und die unidirektionale Kupplung 17, die als Ratsche ausgestaltet ist wird, sind drehfest miteinander verbunden, wobei die unidirektionale Kupplung 17 und das Rotationsglied 7 mittels eines entweder von dem Rotationsglied 7 oder von der unidirektionalen Kupplung 17 gebildeten Elastizitätsmittels, wie zum Beispiel eines Rasterarms ineinandergreifen, wobei das Teil, welches den Rasterarm nicht bildet, vorzugsweise eine Verzahnung aufweist, über die der Rasterarm rasten kann, wobei die Verzahnung zum Beispiel sägezahnförmige Zähne aufweist, welche bewirken, dass das Elastizitätsmittel nur in eine Drehrichtung über die Zähne gleiten kann und in die Gegenrichtung gegen ein Übergleiten der Zähne gesperrt wird. Alternativ kann die unidirektionale Kupplung 17 drehfest mit dem Rotationsglied 7 verbunden sein, wobei die unidirektionale Kupplung 17 dann mittels des Elastizitätsmittels und der Verzahnung mit der Innenhülse 1h verbunden sein könnte.

Das Rotationsglied 7 weist eine zweite Kupplungsstruktur 7a in der Gestalt einer Innenverzahnung, die insbesondere am proximalen Ende des Rotationsglieds 7 gebildet ist, auf.

An dem proximalen Ende des Gehäuses 1, insbesondere der Außenhülse 1g ist eine Außenhülse 2a eines Dosiseinstellglieds 2 drehbar und axialfest befestigt. Die Außenhülse 2a weist an ihrem Innenumfang eine um die Längsachse L umlaufende Ringnut auf, in welche ein am Außenumfang der Außenhülse 1g gebildetes Eingriffsglied eingreift, wodurch die Außenhülse 2a und somit das Dosiseinstellglied 2 drehbar und axialfest an dem Gehäuse 1 befestigt ist. An ihrem Außenumfang weist die Außenhülse 2a eine strukturierte Oberfläche auf, um den Benutzer der Vorrichtung das Verdrehen der Außenhülse 2a relativ zu dem Gehäuse 1 zu vereinfachen. Das Dosiseinstellglied 2 bildet das proximale Ende der Antriebs- und Dosiervorrichtung bzw. der Injektionsvorrichtung. Das proximale Ende der Außenhülse 2a ist geschlossen. Die Außenhülse 2a kann wie hier gezeigt topfförmig sein, wobei der Boden des Topfs das proximale Ende der Injektionsvorrichtung und die Seitenwand des Topfs die Außenhülse 2a bildet.

Das Dosiseinstellglied 2 weist eine Innenhülse 2c auf, wobei die Innenhülse 2c und die Außenhülse 2a zumindest drehfest und optional axial verschiebbar oder axialfest ineinandergreifen. Die Innenhülse 2c kann mit der Außenhülse 2a optional einteilig sein. Die Innenhülse 2c ist über einen Kragen in ihrem proximalen Bereich mit der Außenhülse 2a verbunden. Der Kragen weist mindestens einen Durchbruch 2d, hier zwei Durchbrüche 2d auf, wobei sich durch den mindestens einen Durchbruch 2d ein nach distal von einem Federgehäuse 15 abragender Arm 15a erstreckt. Ein die Innenhülse 2 umgebender Stoppring 13 und die Innenhülse 2 greifen verdrehfest und axial verschiebbar ineinander. Hierfür weist die Innenhülse 2c an ihrem Außenumfang mindestens eine Längsrippe 2e auf, die sich entlang der Längsachse L erstreckt. Das ringförmige Stoppelement 13, insbesondere Stoppring greift in die Längsrippen 2e ein.

Das Gehäuse 1, insbesondere die Außenhülse 1g weist zum Beispiel an ihrem proximalen Ende eine umlaufende Innenverzahnung 1f auf, in die ein Rastglied 2f, welches über einen Arm federnd an der Innenhülse 2c gebildet ist, federnd eingreift. Mittels Verdrehung des Dosiseinstellglieds 2 rastet das Rastglied 2f über die Zähne der Innenverzahnung 1f, wodurch ein akustisches und/oder taktiles Signal erzeugt wird, welches die Dosiseinstellung signalisiert. Ferner wird dadurch bewirkt, dass das Dosiseinstellglied 2 in diskreten Drehwinkelposition einrastet. Insbesondere kann die Verzahnung so ausgebildet sein, insbesondere die Zahnteilung, dass der Winkelabstand zwischen zwei diskreten Drehwinkelpositionen dem Winkelabstand zwischen zwei benachbarten Skalenwerten einer Skala 10b eines Dosisanzeigeglieds 10 oder einer Dosis von einem IU oder einer (1) oder der kleinsten auf der Skala 10b angegebenen Einheit entspricht.

An ihrem distalen Ende weist die Innenhülse 2c eine dritte Kupplungsstruktur 2b, die als Innenverzahnung ausgestaltet ist, auf. Die Innenhülse 2c umgibt ein hülsenförmiges Antriebsglied 4, welches eine vierte Kupplungsstruktur 4b in der Gestalt einer Außenverzahnung aufweist, wobei die vierte Kupplungsstruktur 4b und die dritte Kupplungsstruktur 2b eine zweite Kupplung 2b, 4b bilden, die bei einem unbetätigten Betätigungsglied 8 eingekuppelt ist, d. h., dass die dritte Kupplungsstruktur 2b und die vierte Kupplungsstruktur 4b verdrehfest ineinandergreifen, wodurch eine Drehung des Dosiseinstellglieds 2 über die zweite Kupplung 2b, 4b auf das Antriebsglied 4 übertragen werde kann.

Das Antriebsglied 4 weist insbesondere an seinem proximalen Ende eine erste Kupplungsstruktur 4a in der Gestalt einer Außenverzahnung auf. Das Rotationsglied 7 weist ans seinen proximalen Ende eine zweite Kupplungsstruktur 7a in der Gestalt einer Innenverzahnung auf. Die erste Kupplungsstruktur 4a und die zweite Kupplungsstruktur 7a bilden eine erste Kupplung 4a, 7a, die bei unbetätigtem Betätigungsglied 8 ausgekuppelt ist, d. h., dass die erste Kupplungsstruktur 4a und die zweite Kupplungsstruktur 7a außer Eingriff sind, wodurch das Antriebsglied 4 relativ zu dem Rotationsglied 7 verdreht werden kann.

Das Dosiseinstellglied 2, insbesondere die Außenhülse 2a nimmt ein Federgehäuse 15 auf, welches axial verschiebbar und drehfest mit dem Dosiseinstellglied 2 verbunden ist. Einerseits greifen die Außenhülse 2a und das Federgehäuse 15 drehfest und axial verschiebbar ineinander, andererseits bewirken die sich durch die Durchbrüche 2d erstreckenden Arme 15a des Federgehäuse 15 eine drehfeste und axial verschiebbare Verbindung zwischen dem Federgehäuse 15 und der Innenhülse 2c. Eine Drehung des Dosiseinstellglieds 2 bewirkt allgemein ausgedrückt eine Drehung des Federgehäuses 15.

Das Federgehäuse 15 greift axialfest und drehbar in das Antriebsglied 4 ein. Zwischen dem Federgehäuse 15 und dem Dosiseinstellglied 2 ist eine als Druckfeder wirkende und als Wendelfeder ausgestaltete Rücksetzfeder 9 angeordnet. Insbesondere stützt sich die Rücksetzfeder 9 mit ihrem distalen Ende an der Innenhülse 2c und mit ihrem proximalen Ende an dem Federgehäuse 15 ab, wodurch sie das Federgehäuse 15 und somit auch das Antriebsglied 4 in die proximale Richtung drückt. Die Rücksetzfeder 9 bewirkt, dass die erste Kupplung 4a, 7a ausgekuppelt und die zweite Kupplung 2b, 4b eingekuppelt sind, wenn das Betätigungsglied 8 unbetätigt ist. Durch Verschieben des Federgehäuses 15 und somit des Antriebsglieds 4 in die distale Richtung wird zunächst die erste Kupplung 4a, 7a eingekuppelt und anschließend die zweite Kupplung 2b, 4b ausgekuppelt. Das Federgehäuse 15 weist eine sechste Kupplungsstruktur 15b auf, die in dem gezeigten Beispiel an dem Arm 15a gebildet ist, aber auch an einer anderen Stelle des Federgehäuses 15 gebildet sein könnte. Die sechste Kupplungsstruktur 15b kann eine Außenverzahnung aufweisen. Das Gehäuse 1 weist zum Beispiel an seinem proximalen Ende eine fünfte Kupplungsstruktur, wie zum Beispiel in der Gestalt einer Innenverzahnung, auf. Die fünfte Kupplungsstruktur wird in dem gezeigten Beispiel von der Innenverzahnung 1f gebildet, könnte grundsätzlich aber auch separat von der Innenverzahnung 1f gebildet sein.

Die fünfte Kupplungsstruktur 1f und die sechste Kupplungsstruktur 15b bilden eine dritte Kupplung 1f, 15b, die ausgekuppelt ist, wenn das Betätigungsglied 8 unbetätigt ist und eingekuppelt ist, wenn das Betätigungsglied 8 betätigt ist. Die dritte Kupplung 1f, 15b ist in Bezug auf die zweite Kupplung 2b, 4b so ausgestaltet, dass sie beim Verschieben des Betätigungsglieds 8 aus der unbetätigten Position in die betätigte Position einkuppelt bevor die zweite Kupplung 2b, 4b auskuppelt. In Bezug auf die erste Kupplung 4a, 7a kann die dritte Kupplung 1f, 15b so angeordnet sein, dass sie beim Verschieben des Betätigungsglieds 8 aus der unbetätigten Position vor, gleichzeitig mit oder nach dem Einkuppeln der ersten Kupplung 4a, 7a eingekuppelt. Die Rücksetzfeder 9 bewirkt, dass die dritte Kupplung 1f, 15b ausgekuppelt ist, wenn das Betätigungsglied 8 unbetätigt ist.

Das Federgehäuse 15, insbesondere ein hülsenförmiger Abschnitt des Federgehäuses 15 umgibt eine Antriebsfeder 5, die in diesem Beispiel als Dreh- oder Torsionsfeder wirkt. Die Antriebsfeder 5 ist aus einem bandförmigen Material, wie zum Beispiel Federstahl, spiralförmig gewickelt und umgibt einen Abschnitt des Antriebsglieds 4, der insbesondere zapfenförmig ist. Ein Ende der Antriebsfeder 5 ist verdrehfest mit dem Federgehäuse 15, insbesondere mit dessen hülsenförmigen Abschnitt verbunden. Das andere Ende der Antriebsfeder 5 ist mit dem Antriebsglied 4, insbesondere seinem zapfenförmigen Abschnitt verdrehfest verbunden. Die Antriebsfeder 5 ist so stark vorgespannt, dass die in ihr gespeicherte Federenergie ausreicht, das in dem Produktbehälter 6 enthaltene Produkt vollständig, insbesondere in mehreren Teilausschüttungen auszuschütten, ohne erneut vorgespannt werden zu müssen. Die Antriebsfeder 5 beaufschlagt das Antriebsglied 4 somit mit einem relativ zu dem Federgehäuse 15 in die zweite Drehrichtung wirkenden Drehmoment, welches versucht, das Antriebsglied 4 relativ zu dem Federgehäuse 15 bzw. zu dem Gehäuse 1 in die zweite Drehrichtung zu verdrehen. Aufgrund der eingekuppelten zweiten Kupplung 2b, 4b ist eine Verdrehung des Antriebsglieds 4 relativ zu dem Federgehäuse 15 blockiert, so dass aufgrund der Antriebsfeder 5 noch keine Verdrehung stattfinden kann.

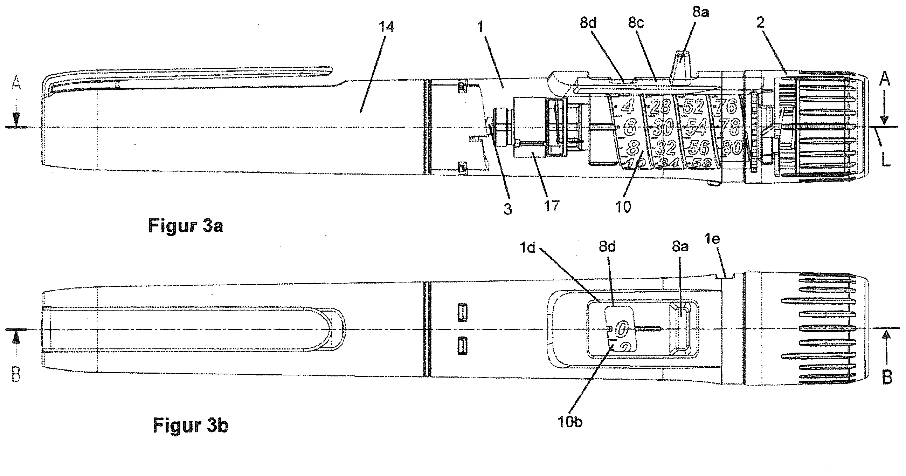

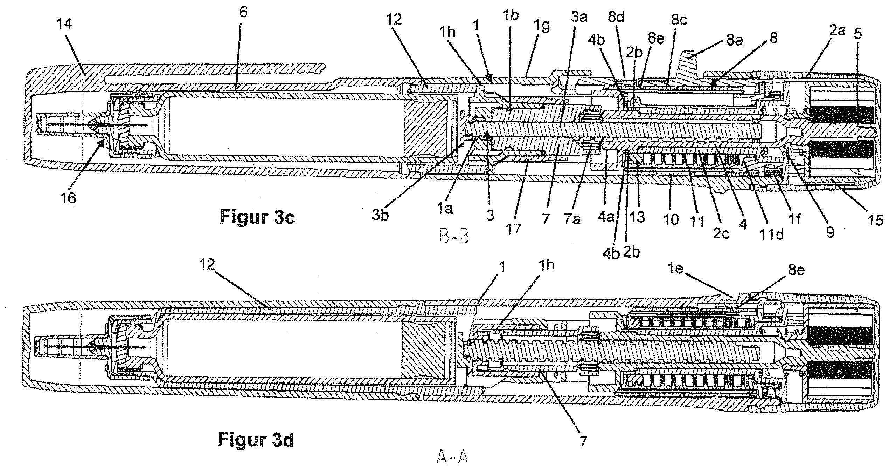

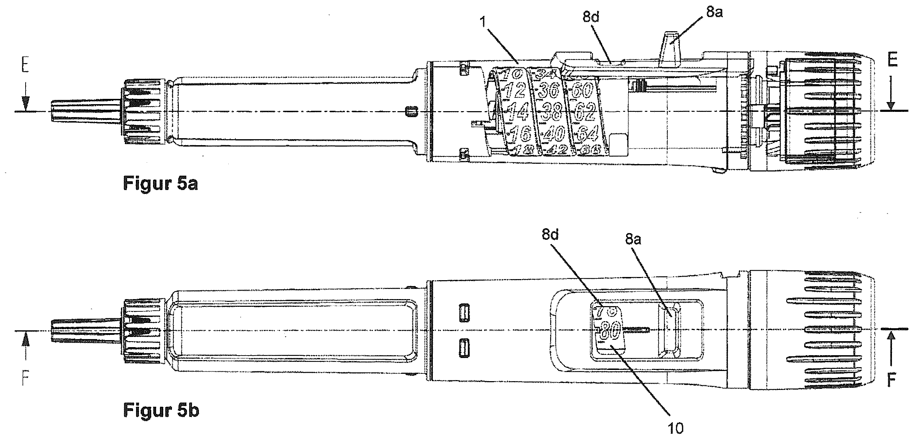

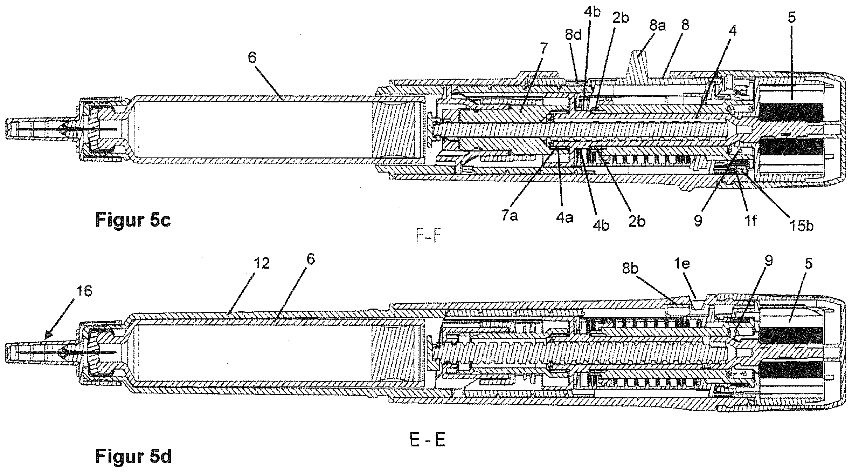

Die Antriebs- und Dosiervorrichtung weist ein relativ zu dem Gehäuse 1 verdrehfest und entlang der Längsachse L verschiebbares Betätigungsglied 8 auf, welches zum Auslösen der Ausschüttung einer eingestellten Produktdosis aus einer unbetätigten Position (Figur 3c) entlang der Längsachse L in eine betätigte Position (Figur 5c) verschiebbar ist. Das Gehäuse 1, insbesondere die Außenhülse 1g bildet eine Führung, welche das Betätigungsglied 8 drehfest und entlang der Längsachse L führt. Insbesondere wird ein Hauptabschnitt 8c des Betätigungsglieds 8 von dem Gehäuse 1 längsgefühn. Der Hauptabschnitt 8c ist um die Längsachse L gekrümmt und erstreckt sich nur teilweise über den Umfang des Gehäuses 1. Der Hauptabschnitt 8c ist innerhalb der Außenhülse 1g angeordnet. Die Außenhülse 1g weist einen Durchbruch auf, durch den sich eine an dem Hauptabschnitt 8c gebildete und nach außen erstreckende Abragung 8a erstreckt. Die Abragung 8a ragt über die Außenfläche der Außenhülse 1g, wodurch der Benutzer die Abragung 8a mit zum Beispiel einem Finger aus der unbetätigten Position in die betätigte Position verschieben kann. Die Abragung 8a kann auch als Betätigungsabschnitt bezeichnet werden, da sie als solcher dient. Das Betätigungsglied 8, insbesondere der Hauptabschnitt 8c weist distal des Betätigungsabschnitts 8a eine Zeigeeinrichtung 8d auf, die ein als Durchbruch gestaltetes Fenster umfasst. Die Zeigeeinrichtung 8d kann zusätzlich einen Pfeil oder eine Markierung, wie zum Beispiel in der gezeigten Ausführungsform eine in das Fenster ragende Nase aufweisen, welche aus mehreren im Fenster 8d gezeigten Skalenwerten der Skala 10b des Dosisanzeigeglieds 10 auf einen bestimmten, nämlich den ausgewählten Skalenwert zeigt, der der eingestellten Produktdosis entspricht.

Der Hauptabschnitt 8c des Betätigungsglieds 8 weist ein Innengewinde 8e oder zumindest einen Innengewindeabschnitt auf, der in ein Außengewinde 10a am Außenumfang des als Dosisanzeigetrommel gebildeten Dosisanzeigeglieds 10 eingreift. Das Dosisanzeigeglied 10 weist an seinem Außenumfang eine helixförmig umlaufende Dosisskala 10b auf, die eine Vielzahl aneinandergereihte Skalenwerte, welche hier in Zweierschritten Zahlen und in Einserschritten z.B. strichförmige Markierungen umfasst. Die Steigung der sich helixförmig erstreckenden Dosisskala 10b entspricht der Steigung des Gewindes 10a. Durch Verdrehen des Dosisanzeigeglieds 10 relativ zu dem Betätigungsglied 8 wird das Dosisanzeigeglied 10 an den Betätigungsglied 8 entlanggeschraubt. Hierdurch werden die Dosisskala 10b bzw. die Skalenwerte der Dosisskala 10b unter der Zeigeeinrichtung 8d hindurchbewegt, wodurch die momentan eingestellte Produktdosis von dem Benutzer durch das Fenster 8d hindurch abgelesen werden kann.

Das Dosisanzeigeglied 10 ist zwischen einer Position, in der in der Zeigeeinrichtung 8d die Dosis Null angezeigt wird (Nulldosisposition) und einer Maximaldosisposition, der in der Zeigeeinrichtung 8d der maximal mit einer Dosierung ausschüttbare Dosiswert angezeigt wird, wie zum Beispiel 80 IU, hin und her schraubbar, insbesondere durch Hin- und Herdrehen des Dosiseinstellglieds 2 in die erste und zweite Drehrichtung. Das Dosisanzeigeglied 10 weist einen in Umfangsrichtung wirkenden Nulldosisanschlag 10d und einen in Umfangsrichtung wirkenden Maximaldosisanschlag 10c auf. In der Nulldosisposition des Dosiseinstellglieds 10 kann der Nulldosisanschlag 10d an einem von der Vorrichtung gebildeten Nulldosisgegenanschlag 11c anschlagen, der in dem gezeigten Beispiel von einer Verschiebehülse 11 gebildet wird. Die Verschiebehülse 11 ist axial verschiebbar und drehfest mit dem Gehäuse 1, insbesondere der Außenhülse 1g verbunden. Hierfür kann die Verschiebehülse 11b eine Eingriffsstruktur 11b aufweisen, welche in eine Eingriffsstruktur, wie zum Beispiel die Verzahnung 1f oder eine zusätzliche Verzahnung, am Innenumfang des Gehäuses 1, insbesondere der Außenhülse 1g eingreift.

Ein Maximaldosisgegenanschlag 12a, an dem der Maximaldosisanschlag 10c in der Maximaldosisposition anschlagen kann, kann von einem Produktbehälterhalter 12, der vorzugsweise dreh- und axialfest mit dem Gehäuse 1, insbesondere der Außenhülse 1g verbunden, wie zum Beispiel verschnappt ist, gebildet werden.

Das Dosisanzeigeglied 10 und das Antriebsglied 4 greifen verdrehfest und axial verschiebbar ineinander, wodurch eine Drehung des Antriebsglieds 4 eine Drehung des Dosisanzeigeglieds 10 bewirkt. Hierfür ist am Innenumfang des Dosisanzeigeglieds 10 mindestens eine Längsftihrung 10f gebildet, die in einem Eingriff mit dem Antriebsglied 4 ist.

Die Verschiebehülse 11, die auch als Verschiebeglied 11 bezeichnet werden kann, und das Betätigungsglied 8 greifen axialfest ineinander, wodurch die Verschiebehülse 11 die Axialbewegungen des Betätigungsglieds 8 entlang der Längsachse L mitmacht. Das freie Ende der Arme 15a ist mit der Verschiebehülse 11 axialfest verschnappt, wodurch das Federgehäuse 15 die Axialbewegung der Verschiebehülse 11 mitmacht. Die Verschiebehülse 11 weist eine Eingriffsstruktur 11b auf, welche in eine Eingriffsgegenstruktur des Gehäuse 1, insbesondere der Außenhülse 1g verdrehfest und axial verschiebbar eingreift, wodurch die Verschiebehülse 11 in Bezug auf das Gehäuse 1 verdrehfest und axial verschiebbar ist.

Der Stoppring 13 weist ein Außengewinde 13 auf, welche in das Innengewinde 11a des Verschiebeglieds 11 eingreift, wodurch das Stoppelement 13 an der Verschiebehülse 11 entlang schraubbar ist. An seinem Innenumfang weist das Stoppelement 13 mindestens eine Längsführung 13b auf, welche in eine entsprechende Längsführung des Dosiseinstellglieds 2, insbesondere deren Innenhülse 2c eingreift, wodurch das Stoppelement 13 in Bezug auf das Dosiseinstellglied 2 drehfest und axial verschiebbar ist. Das Stoppelement 13 ist Teil einer Einrichtung, die verhindert, dass eine Dosis eingestellt werden kann, welche die in dem Produktbehälter enthaltene oder ausschüttbare Produktmenge übersteigt. Die Einrichtung kommt nur dann zu tragen, wenn die in dem Produktbehälter 6 enthaltene oder ausschüttbare Produktmenge kleiner als die mit der Antriebs- und Dosiervorrichtung einstellbare Maximaldosis ist. Das Stoppelement 13 weist einen Stoppanschlag 13c auf, der an einem Stoppgegenanschlag 11d der von der Verschiebehülse 11 gebildet wird, anschlagen kann.

Die Antriebs- und Dosiervorrichtung der ersten Ausführungsform weist optional eine Einrichtung auf, die anzeigt, wenn das Betätigungsglied 8 betätigt ist (Betätigungs-Markierung) und/oder das Dosisanzeigeglied 10 in seiner Nulldosisposition ist (Nulldosis-Markierung), unabhängig von der Zeigeeinrichtung 8d, welche die aktuell eingestellte Dosis anzeigt. Für die Betätigungs-Markierung weist der Hauptabschnitt 8c des Betätigungsglieds 8 einen sich in Umfangsrichtung von dem Hauptabschnitt 8c erstreckenden Flügel auf, an dem eine Markierung 8b angeordnet ist. Alternativ kann der Flügel distal oder bevorzugt proximal von der Markierung einen transparenten Bereich (nicht gezeigt) aufweisen. In der unbetätigten Position des Dosiseinstellglieds 8 ist die Markierung so unterhalb eines von dem Gehäuse 1, insbesondere der Außenhülse 1g gebildeten Sichtfensters 1e angeordnet, dass die Markierung von dem Benutzer über das Sichtfenster 1e abgelesen werden kann. Durch das Verschieben des Betätigungsglieds 8 aus der unbetätigten Position in die betätigte Position wird die Markierung 8b zusammen mit dem Betätigungsglied 8 verschoben, wobei die Markierung 8b aus der Position unterhalb des Sichtfensters 1e verschoben wird, so dass sie nicht mehr ablesbar ist. Dabei gelangt der transparente Bereich in das Sichtfenster 1e und schützt das Fenster insbesondere die Vorrichtung vor äusserlichen Eingriffen. Die Markierung 8b wird von einem Abschnitt der Außenhülse 1g abgedeckt. Das Sichtfenster 1e ist winkelversetzt und proximal des Betätigungsabschnitts 8a angeordnet, so dass der Benutzer der Vorrichtung bei Betätigung des Betätigungsabschnitts 8a das Sichtfenster 1e möglichst nicht verdeckt. Hierdurch wird dem Benutzer zusätzlich ermöglicht, visuell festzustellen, ob das Betätigungsglied 8 betätigt ist.

Für die Nulldosis-Markierung weist das Dosisanzeigeglied 10 eine von der Dosisanzeigeskala 10b abgesetzte Markierung 10e auf, welche in der Nulldosisposition des Dosisanzeigeglieds 10 unter dem Sichtfenster 1e angeordnet ist, wodurch dem Benutzer der Vorrichtung bei betätigtem Betätigungsglied 8 visuell angezeigt wird, ob das Dosisanzeigeglied 10 in seiner Nulldosisposition ist, unabhängig von dem in der Zeigeeinrichtung 8d angezeigten Skalenwert. Durch das Verschieben des Betätigungsglieds 8 in die betätigte Position wird die Markierung 8b aus dem Sichtfenster 1e verschoben und der transparente Bereich der Markierung 8b schützt das Sichtfenster 1e. Wenn eine Nulldosis eingestellt wurde oder wenn die Produktausschüttung erfolgt ist, erscheint in dem Sichtfenster 1e, unterhalb des transparenten Bereichs, die Markierung 10e. Hierdurch erhält der Benutzer eine zusätzliche Anzeige darüber, ob die Produktausschüttung vollständig beendet wurde.

Alternativ kann die Markierung 10e unterhalb eines Fensters (nicht dargestellt) des Dosiseinstellglieds 2a angeordnet sein, so dass bei einer Nulldosiseinstellung bzw. bei einer erfolgten Produktausschüttung in dem Fenster des Dosiseinstellglieds 2a die Markierung 10e erscheint und dem Benutzer mittgeteilt wird, dass die Produktausschüttung erfolgt ist.

Grundsätzlich ist es möglich, für die Markierung 10e ein von dem Sichtfenster 1e separates Sichtfenster (nicht dargestellt) vorzusehen.

In den Figuren 3a bis 3d wird die Injektionsvorrichtung in einem Auslieferungszustand oder einem Ausgangszustand gezeigt. Zum Verwenden der Vorrichtung wird die Schutzkappe 14, welche den Produktbehälterhalter 12 umgibt, der einen Produktbehälter 6, der hier in der Gestalt einer Karpule dargestellt ist, und eine am distalen Ende des Produktbehälterhalters 12 angebrachte oder anbringbare Nadeleinheit 16 abdeckt, angenommen. Das Betätigungsglied 8 wird mittels der Rücksetzfeder 9 in seiner unbetätigten Position gehalten. In dem Sichtfenster 1e wird die Markierung 8b angezeigt, wodurch zusätzlich hervorgeht, dass das Betätigungsglied 8 unbetätigt ist. In der Zeigeeinrichtung 8d wird die Dosis Null angezeigt.

Für die Einstellung der auszuschüttenden Produktdosis wird zur Dosiserhöhung das Dosiseinstellglied 2 relativ zu dem Gehäuse I in eine erste Drehrichtung gedreht, wobei die in der Zeigeeinrichtung 8d angezeigten Skalenwerte vorwärtszählen. Zur Verringerung der Dosis oder zur Dosiskorrektur wird das Dosiseinstellglied 2 in die der ersten Drehrichtung gegengesetzte Drehrichtung, nämlich in eine zweite Drehrichtung gedreht, wodurch die in der Zeigeeinrichtung 8 gezeigten Skalenwerte rückwärtszählen. Die Drehung des Dosiseinstellglieds 2 wird über die geschlossene zweite Kupplung 2b, 4b auf das Antriebsglied 4 und von dem Antriebsglied 4 auf das Dosisanzeigeglied 10 übertragen, welches sich bei der Dosiserhöhung in die distale Richtung, nämlich zu dem Maximaldosisgegenanschlag 12a hin schraubt und bei Drehung des Dosiseinstellglieds 2 in die zweite Drehrichtung in die proximale Richtung, nämlich zu dem Nulldosisgegenanschlag 11c hin schraubt. Alternativ kann sich das Dosisanzeigeglied 10 bei der Dosiserhöhung, in die proximale Richtung zum Maximaldosisgegenanschlag 12a und bei Dosisverringerung, in die distale Richtung zu dem Nulldosisgegenanschlag 11c hin schrauben. Da sich bei unbetätigtem Betätigungsglied 8 das Federgehäuse 15 und das Antriebsglied 4 mit dem Dosiseinstellglied 2 mitdrehen und mithin nicht zueinander verdreht werden, wird die Antriebsfeder 5 weder gespannt noch entspannt. Aufgrund der ausgekuppelten ersten Kupplung 4a, 7a kann sich das Antriebsglied 4 relativ zu dem Rotationsglied 7 drehen. Da sich die Innenhülse 2c mit der Außenhülse 2a des Dosiseinstellglieds 2 während der Dosiseinstellung mitdreht, wird das Stoppelement 13 bei der Dosiserhöhung oder Drehung des Dosiseinstellglieds 2 in die erste Drehrichtung zu dem Stoppgegenanschlag 11d hin geschraubt und bei der Drehung des Dosiseinstellglieds 2 in die zweite Drehrichtung von dem Stoppanschlag 11d weg geschraubt. Der Abstand entlang der helixförmigen Kurve, den der Stoppanschlag 13c zum Stoppgegenanschlag 11c aufweist, ist proportional zu der in dem Produktbehälter 6 enthaltenen oder ausschüttbare Produktmenge. Wenn die gewünschte Dosis eingestellt wurde, kann das Betätigungsglied 8 betätigt werden.

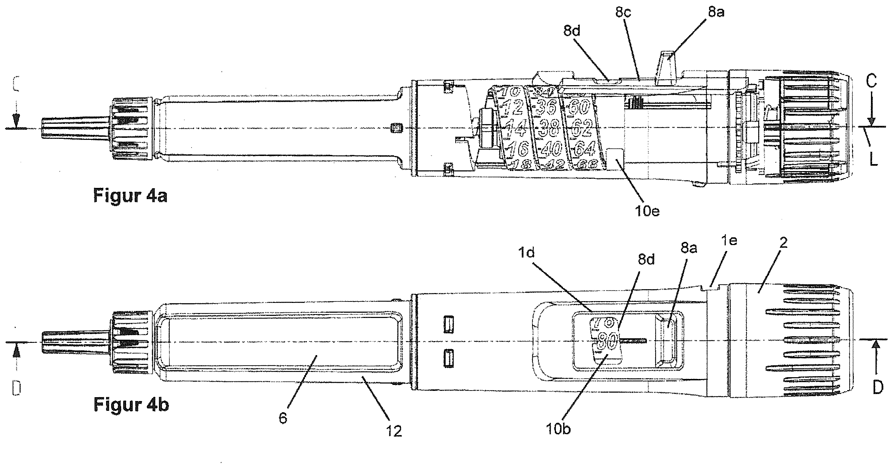

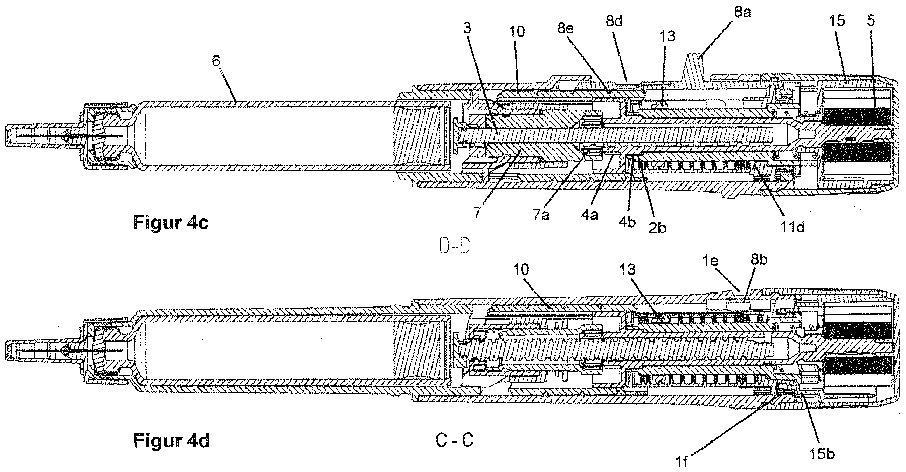

In den Figuren 4a-4d wird die Injektionsvorrichtung in einem maximal aufdosierten Zustand gezeigt, bei dem in der Zeigeeinrichtung 8d die maximal einstellbare Dosis, die in diesem Beispiel 80 IU beträgt, angezeigt wird. Da die in dem Produktbehälter 6 enthaltene Produktmenge größer als die maximal mit der Antriebs- und Dosiervorrichtung einstellbare Produktdosis ist, schlägt das Stoppelement 13 noch nicht an dem Stoppgegenanschlag 11c an. Der Maximaldosisanschlag 10c liegt z.B. an dem Maximaldosisgegenanschlag 12a an. Zum Ausschütten der eingestellten Produktdosis wird der Betätigungsabschnitt 8a in die distale Richtung verschoben, wodurch das Betätigungsglied 8 aus der unbetätigten Position in die distale Richtung in die betätigte Position verschoben wird. Bei der Verschiebung des Betätigungsglieds 8 in die distale Richtung wird das Dosisanzeigeglied 10 ebenfalls in die distale Richtung mitgenommen, wobei sich die Zeigeeinrichtung 8d zwar relativ zu dem Gehäuse 1 aber nicht relativ zu dem Dosisanzeigeglied 10 verschiebt. Durch die Verschiebung des Betätigungsglieds 8 wird die Verschiebehülse 11 mitgenommen, welche ihrerseits das Federgehäuse 15 in die distale Richtung mitnimmt, wobei das Federgehäuse 15 das Antriebsglied 4 in die distale Richtung mitnimmt. Die Verschiebung des Antriebsglied 4 und des Federgehäuses 15 in die distale Richtung bewirken, dass die erste Kupplung 4a, 7a zunächst eingekuppelt und anschließend die zweite Kupplung 2b, 4b ausgekuppelt und die dritte Kupplung 1f und 15b eingekuppelt werden. Ferner wird durch das Verschieben des Betätigungsglieds 8 bewirkt, dass die Markierung 8b aus dem Sichtfenster 1e verschoben . Alternativ kann der transparente Bereich (nicht gezeigt) in das Sichtfenster 1e verschoben werden.

Aufgrund der ausgekuppelten zweiten Kupplung 2b, 4b ist das Antriebsglied 4 nun relativ zu dem Dosiseinstellglied 2 drehbar, wodurch die Antriebsfeder 5 das Antriebsglied 4 relativ zu dem Dosiseinstellglied 2 und/oder dem Gehäuse 1 in die zweite Drehrichtung verdreht. Durch die Drehung des Antriebsglieds 4 in die zweite Drehrichtung, wenn das Betätigungsglied 8 betätigt ist, werden die Innenhülse 2c und die Verschiebehülse 11 relativ zueinander nicht verdreht, wodurch das Stoppelement 13 in Bezug auf den Stoppgegenanschlag 11d seine Position beibehält oder nicht verändert. Aufgrund der geschlossenen ersten Kupplung 4a, 7a wird das Rotationsglied 7 von dem Antriebsglied 4 in die zweite Drehrichtung mitgedreht, wodurch das Rotationsglied 7 das Abtriebsglied 3 in die zweite Drehrichtung dreht, wodurch sich das Abtriebsglied 3 aufgrund des Gewindeeingriffs mit dem Gehäuse 1 in die distale Richtung schraubt und dabei den in dem Produktbehälter 6 verschiebbar aufgenommene Kolben mitnimmt und in die distale Richtung verschiebt, wodurch das in dem Produktbehälter 6 enthaltene Produkt über die Nadel ausgegeben wird.

Wenn während der Produktausschüttung das Betätigungsglied 8 losgelassen wird, wird es durch die Rücksetzfeder 9 aus der betätigten Position in die unbetätigte Position zurückgesetzt, wobei die zweite Kupplung 2b, 4b eingekuppelt und die dritte Kupplung 1f, 15b eingekuppelt und die erste Kupplung 4a, 7a ausgekuppelt werden. Wenn die Produktausschüttung noch nicht vollständig abgeschlossen ist, wird in der Zeigeeinrichtung 8d die Dosis angezeigt, welche bis zur vollständigen Produktausschüttung noch ausgeschüttet werden müsste.

Wenn das Betätigungsglied 8 bis zum Ende der Produktausschüttung, das erreicht wird, wenn der Nulldosisanschlag 10d an den Nulldosisgegenanschlag 11c anschlägt, in der betätigten Position gehalten wird, erscheint die Markierung 10e in dem Sichtfenster 1e, wodurch das Ende der Produktausschüttung visuell angezeigt wird. Alternativ kann die Markierung 10e unterhalb des transparenten Bereichs (nicht gezeigt) oder an einem anderen beliebigen Ort der Antriebs- und Dosiervorrichtung erscheinen. Wenn der Betätigungsabschnitt 8a losgelassen wird, wird das Betätigungsglied 8 aufgrund der Rücksetzfeder 9 in die unbetätigte Position zurückgesetzt, wobei die Markierung 8b in dem Sichtfenster 1e erscheint.

Nun können neue Dosiseinstellungen und Produktausschüttungen entsprechend der oben genannten Funktion ausgeführt werden.

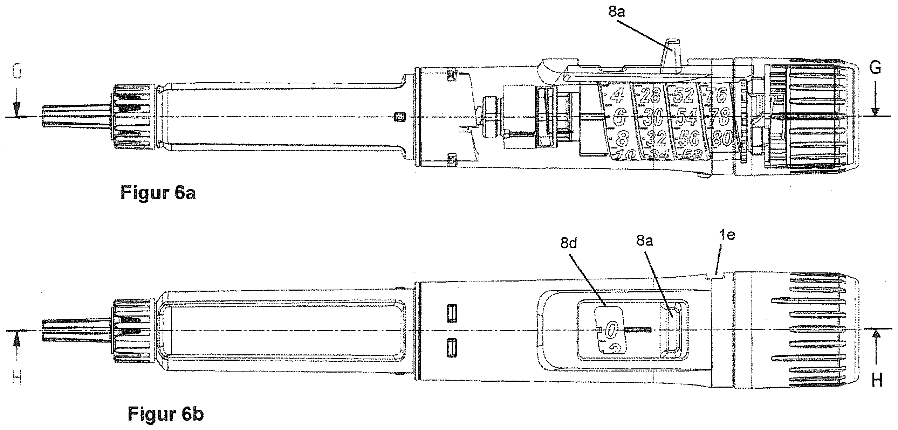

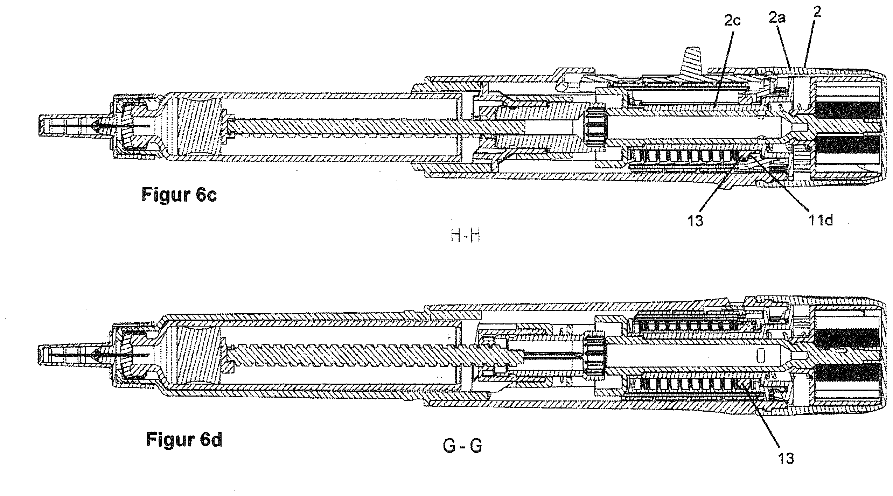

In den Figuren 6a bis 6d wird die Vorrichtung nach dem vollständigen Ausschütten der in dem Produktbehälter 6 enthaltenen oder ausschüttbare Produktmenge gezeigt, wobei das Stoppelement 13 mit seinem Stoppanschlag 13c an dem Stoppgegenanschlag 11d anschlägt. Der Versuch das Dosiseinstellglied 2 in diesem Zustand relativ zu dem Gehäuse 1 zu verdrehen, bewirkt, dass das Stoppelement 13 gegen den Stoppgegenanschlag 11d gedrückt wird, wodurch eine Drehung der Innenhülse 2c in die erste Drehrichtung blockiert wird und somit auch die Drehung der Außenhülse 2a blockiert wird. Allgemein kann somit das Dosiseinstellglied 2 nicht mehr in die erste Drehrichtung gedreht werden. Eine Drehung des Dosiseinstellglieds 2 in die zweite Drehrichtung, welche eine Verringerung der eingestellten Dosis bewirken würde, wird zwar grundsätzlich von dem Stoppelement 13 zugelassen, jedoch von den aneinander anliegenden Nulldosisanschlag 10d und Nulldosisgegenanschlag 11c verhindert. Somit kann das Dosiseinstellglied 2 in keine der beiden Drehrichtungen gedreht werden. Die Vorrichtung kann nun entsorgt werden.

**Bezugszeichenliste für die erste Ausführungsform**

| | | | |
|---|---|---|---|
| 1 | Gehäuse | 8e | Innengewinde |
| 1a | Innengewinde | 9 | Rücksetzfeder |
| 1b | Abragung | | |
| 1d | Ausnehmung | 10 | Dosisanzeige-glied/Dosisanzeigetrommel |
| 1e | Sichtfenster | | |
| 1f | Innenverzahnung/ fünfte Kupplungsstruktur | 10a | Außengewinde |
| | | 10b | Dosisskala |
| 1g | Außenhülse | 10c | Maximaldosisanschlag |
| 1h | Innenhülse | 10d | Nulldosisanschlag |
| | | 10e | Nulldosis-Markierung |
| 2 | Dosiseinstellglied | 10f | Längsführung |
| 2a | Außenhülse | | |
| 2b | dritte Kupplungsstruktur | 11 | Verschiebehülse |
| 2c | Innenhülse | 11a | Innengewinde |
| 2d | Durchbruch | 11b | Eingriffsstruktur |
| 2e | Längsrippe | 11c | Nulldosisgegenanschlag |
| 2f | Rastglied | 11d | Stoppgegenanschlag |
| 3 | Abtriebsglied | 12 | Produktbehälterhalter |
| 3a | Gewindestange | 12a | Maximaldosisgegenanschlag |
| 3b | Flansch | | |
| 3c | Außengewinde | 13 | Stoppelement/Stoppring |
| 3d | Führungsnut | 13a | Außengewinde |
| | | 13b | Längsführung |
| 4 | Antriebsglied | 13c | Stoppanschlag |
| 4a | erste Kupplungsstruktur | | |
| 4b | vierte Kupplungsstruktur | 14 | Kappe |
| 5 | Antriebsfeder | 15 | Federgehäuse |
| 6 | Produktbehälter | 15a | Arm |
| | | 15b | sechste Kupplungsstruktur |
| 7 | Rotationsglied | | |
| 7a | zweite Kupplungsstruktur | 16 | Nadeleinheit |
| 7b | Eingriffsglied | | |
| 7d | Nut | 17 | unidirektionale Kupplung/Ratsche |
| 8 | Betätigungsglied | 4a, 7a | erste Kupplung |
| 8a | Abragung, Betätigungsabschnitt | 2b, 4b | zweite Kupplung |
| 8b | Markierung | 1f, 15b | dritte Kupplung |
| 8c | Hauptabschnitt | | |
| 8d | Fenster/Zeigeeinrichtung | L | Längsachse |

Die in den Figuren 7 bis 11b gezeigte zweite Ausführungsform einer Injektionsvorrichtung mit der erfindungsgemäßen Antriebs- und Dosiervorrichtung umfasst ein längliches Gehäuse 101. An dem vorderen oder distalen Ende des Gehäuses 101 ist ein Produktbehälterhalter 112 angeordnet, in dem ein Produktbehälter 106, in dem gezeigten Beispiel eine Karpule, aufgenommen ist. An dem distalen Ende der Karpule 106 bzw. des Produktbehälterhalters 112 ist eine Nadeleinheit 116 angeordnet, wobei durch die Nadel der Nadeleinheit 116 das in dem Produktbehälter 106 enthaltene vorzugsweise flüssige Produkt oder Medikament ausgegeben werden kann.

Der Produktbehälterhalter 112 und ggf. die Nadeleinheit 116 werden durch eine abnehmbare Kappe 114 abgedeckt, die am Produktbehälterhalter 112 lösbar befestigt, insbesondere verschnappt ist. Die Antriebs- und Dosiervorrichtung bildet zum Beispiel mit dem an die Antriebs- und Dosiervorrichtung angebrachten Produktbehälter 106 und Produktbehälterhalter 112 eine Injektionsvorrichtung.

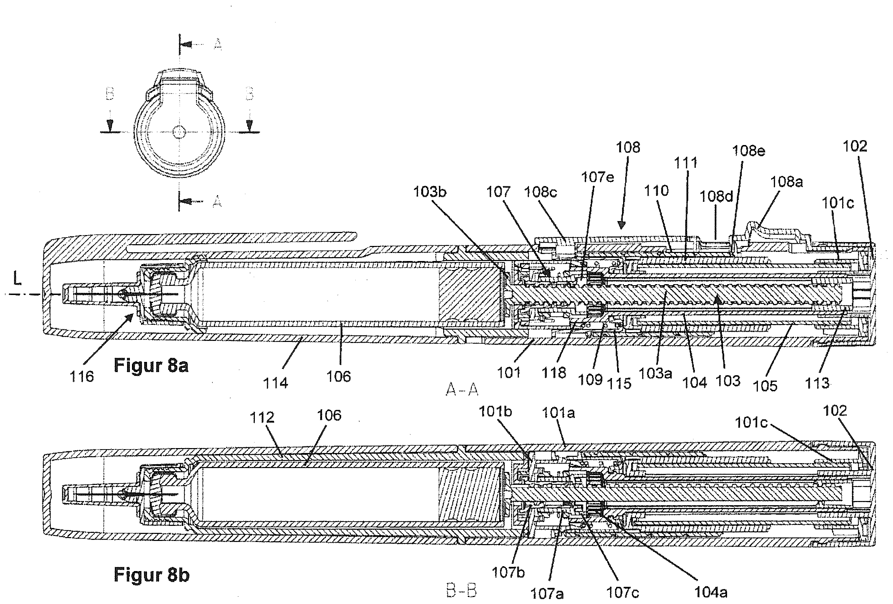

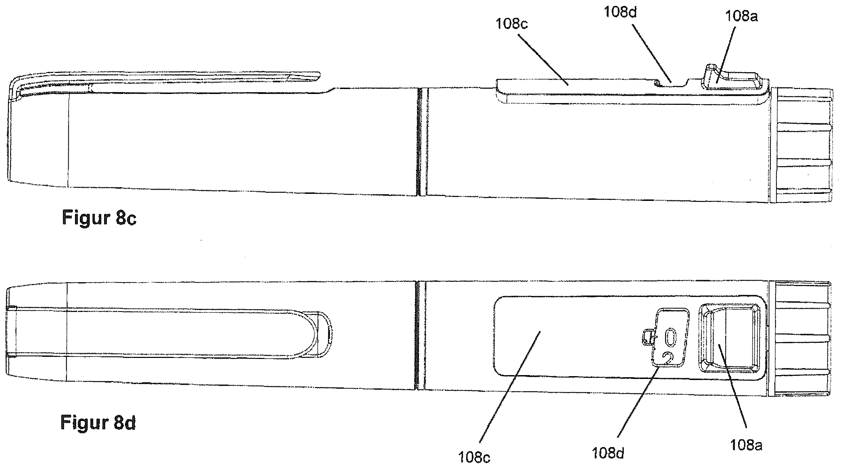

Das Gehäuse 101 ist ein mehrteiliges Gehäuse. Es umfasst eine Außenhülse 101a, einen ersten Gehäuseeinsatz 101b und einen zweiten Gehäuseeinsatz 101c, wie am besten aus Figur 8b ersichtlich ist. Optional könnten die Gehäuseeinsätze 101b, 101c einteilig mit der Außenhülse 101a gebildet sein, so dass das Gehäuse 101 einteilig ist, wobei die Montage der Vorrichtung jedoch mit einem mehrteiligen Gehäuse einfacher ist.

In dem Gehäuse 101 ist ein Rotationsglied 107 drehbar und axialfest angeordnet. Insbesondere greifen das Gehäuse 101 und das Rotationsglied 107 so ineinander, dass das Rotationsglied 107 relativ zu dem Gehäuse 101 drehbar und axialfest ist. Das Rotationsglied 107 könnte grundsätzlich einteilig sein, umfasst aber in diesem Beispiel mehrere Teile, nämlich eine Kupplungshülse 107a und eine Gewindehülse 107b, die miteinander dreh- und axialfest verbunden, insbesondere verschnappt sind. Dadurch können die Teile aus unterschiedlichen Materialien gefertigt sein. Zwischen der Kupplungshülse 107a und der Gewindehülse 107b ist eine Ringnut gebildet, in die der erste Gehäuseeinsatz 101b eingreift, wodurch das Rotationsglied 107 relativ zu dem Gehäuse 101 drehbar und axialfest ist. Das Rotationsglied 107, insbesondere die Gewindehülse 107b weist ein Innengewinde 107d auf, welches in ein Außengewinde 103c einer Gewindestange 103a,die Teil eines Abtriebsglieds 103 ist, eingreift. Die Gewindestange 103a weist mindestens eine Längsführung, insbesondere eine Führungsnut 103d auf, welche das Außengewinde 103c überlagert und sich entlang der Längsachse L der Antriebs- und Dosiervorrichtung erstreckt. Das distale Ende der Gewindestange 103a ist mit einem tellerförmigen Flansch 103b verbunden, wie zum Beispiel einteilig gebildet oder verschnappt, so dass der Flansch 103b zum Beispiel frei drehbar in Bezug auf die Gewindestange 103a ist. Der Flansch 103b und die Gewindestange 103a bilden das Abtriebsglied 103. Das Gehäuse 101 greift in die Führungsnut 103d ein, wodurch das Abtriebsglied 103 relativ zu dem Gehäuse 101 drehfest und axial verschiebbar ist. Um das Abtriebsglied für die Produktausschüttung in die distale Richtung zu bewegen, wodurch der Kolben im Produktbehälter 106 in die distale Richtung verschoben wird, wird das Rotationsglied 107 in eine zweite Drehrichtung gedreht, wodurch die Gewindestange 103 an dem Rotationsglied 107 entlang geschraubt wird und aufgrund der Längsführung mit dem Gehäuse 101 in die distale Richtung verschoben wird.

Zwischen dem Rotationsglied 107 und dem Gehäuse 101 ist eine unidirektionale Kupplung gebildet, welche eine Drehung des Rotationsglieds 107 in die zweite Drehrichtung zulässt und eine Drehung des Rotationsglieds 107 in die erste Drehrichtung, die der zweiten Drehrichtung entgegengesetzten ist, nicht zulässt oder sperrt. Die unidirektionale Kupplung kann auch als Ratsche bezeichnet werden. Die unidirektionale Kupplung umfasst ein Ratschenglied 117, welches verdrehfest mit dem Rotationsglied 107, insbesondere der Kupplungshülse 107a verbunden ist. Das Ratschenglied 117 weist eine stirnseitige Verzahnung 117a auf, die mehrere sägezahnförmige Zähne umfasst und in eine Gegenverzahnung, die mehrere sägezahnförmige Zähne umfasst, des Gehäuses 101, insbesondere des Gehäuseeinsatzes 101b eingreifen. Durch die sägezahnförmigen Zähne lässt sich das Ratschenglied 117 relativ zu dem Gehäuse 101 in die zweite Drehrichtung drehen und relativ zu dem Gehäuse in die erste Drehrichtung nicht drehen. Das Ratschenglied 117 ist relativ zu dem Rotationsglied 107, insbesondere zu der Kupplungshülse 107a axial bewegbar. Das Rotationsglied 107, insbesondere die Kupplungshülse 107a weist eine Außenverzahnung 107f auf, in die eine Innenverzahnung 117b des Ratschenglieds 117 drehfest und axial verschiebbar eingreift. Der Abschnitt des Ratschenglieds 117, der die Verzahnungen 117a und 117b aufweist, stützt sich axial und federnd an der Kupplungshülse 107a des Rotationsglieds 107 ab. Dies kann mittels einer zwischen diesem Abschnitt und der Kupplungshülse 107a gebildeten separaten Druckfeder oder, wie hier gezeigt, mittels eines Federabschnitts 117c, der an den Abschnitt, der die Verzahnungen 117a und 117b bildet, erfolgen. Somit ist der Abschnitt, der die Verzahnungen 117a und 117b bildet, gegen die Federkraft des Federabschnitts 117c in die proximale Richtung verschiebbar.

Wird das Rotationsglied 107 in die zweite Drehrichtung gedreht, rastet die Stirnverzahnung 117a über die Gegenverzahnung des Gehäuses 101 bzw. des ersten Gehäuseeinsatzes 101b, wodurch Klickgeräusche erzeugt werden. Eine Drehung in die Gegenrichtung, nämlich in die erste Drehrichtung wird jedoch verhindert.

An dem proximalen Ende des Gehäuses 101 ist ein Dosiseinstellglied 102 angeordnet, welches in Bezug auf das Gehäuse 101 drehbar und axialfest ist. Das Dosiseinstellglied 102 umgibt das proximale Ende des Gehäuses 101 umfangsseitig. Das Dosiseinstellglied 102 ist an seinem proximalen Ende verschlossen und bildet das proximale Ende der Antriebs- und Dosiervorrichtung. Das Dosiseinstellglied 102 weist an seinem Innenumfang eine Ringnut auf, in die ein Vorsprung des Außenumfangs des Gehäuses 101 eingreift, wodurch das Dosiseinstellglied 102 drehbar und axialfest in Bezug auf das Gehäuse 101 ist. Das Dosiseinstellglied 102 wird für die Einstellung einer Dosis relativ zu dem Gehäuse 101 gedreht, insbesondere für die Erhöhung einer auszuschüttenden Dosis in eine erste Drehrichtung und für die Verringerung oder Ausschüttung der auszuschüttenden Dosis in eine zweite Drehrichtung, die der ersten Drehrichtung entgegengesetzt ist.

In dem Gehäuse 101, insbesondere der Außenhülse 101a, ist ein hülsenförmiges Antriebsglied 104 angeordnet, welches die Gewindestange 103a oder das Abtriebsglied 103 umgibt. Das Antriebsglied 104 ist in Bezug auf das Gehäuse 101 entlang der Längsachse L axial verschiebbar und um die Längsachse L verdrehbar. Das Dosiseinstellglied 102 und das Antriebsglied 104 sind drehfest und axial verschiebbar miteinander verbunden oder greifen drehfest und axial verschiebbar ineinander, wodurch das Antriebsglied 104 die Drehbewegung des Dosiseinstellglieds 102 mitmacht und umgekehrt.

Die Antriebs- und Dosiervorrichtung weist ein hülsenförmiges Dosisanzeigeglied 110 auf, welches als Dosisanzeigetrommel ausgestaltet ist und das Antriebsglied 104 umgibt. Das Dosisanzeigeglied 110 weist an ihrem Außenumfang ein Gewinde 110a und eine Dosisskala 110b auf. Die Dosisskala 110b weist eine Vielzahl von helixförmig aneinandergereihten Skalenwerten auf, die zusammen die helixförmige Dosisskala 110b ergeben, wie bei der ersten Ausführungsform. Die helixförmige Dosisskala 110b und das Gewinde 110a weisen die gleiche Steigung auf.

Die Antriebs- und Dosiervorrichtung weist ein Betätigungsglied 108 (Figuren 8a,b) auf, welches in Bezug auf das Gehäuse 101 um die Längsachse L drehfest und entlang der Längsachse L axial verschiebbar ist. Insbesondere greift das Betätigungsglied 108 so in das Gehäuse 101 ein, dass es drehfest und axial verschiebbar ist. Das Betätigungsglied 108 weist mindestens einen Gewindeabschnitt insbesondere ein Innengewinde 108e auf, der in das Aussengewinde 110a des Dosisanzeigeglieds 110 eingreift, wodurch das Dosisanzeigeglied 110 an dem Betätigungsglied 108 entlang schraubbar ist, wenn das Dosisanzeigeglied 110 um die Längsachse L gedreht wird. Das Dosisanzeigeglied 110 ist drehfest und axial verschiebbar mit dem Antriebsglied 104 verbunden, insbesondere unmittelbar oder mittelbar, wie zum Beispiel über ein hülsenförmiges Zwischenglied 111, welches auch als Zwischenhülse bezeichnet werden kann, wie in dem zweiten Ausführungsbeispiel gezeigt wird. Das Antriebsglied 104 und das Zwischenglied 111 greifen um die Längsachse L drehfest und axial verschiebbar ineinander, wodurch das Zwischenglied 111 die Drehbewegung des Antriebsglieds 104 mitmacht und relativ zu dem Antriebsglied 104 entlang der Längsachse L verschiebbar ist. Das Zwischenglied 111 und das Dosisanzeigeglied 110 greifen um die Längsachse L drehfest und entlang der Längsachse L verschiebbar ineinander, wodurch das Dosisanzeigeglied 110 die Drehbewegung des Zwischenglieds 111 mitmacht und relativ zu dem Zwischenglied 111 entlang der Längsachse L verschiebbar ist. Somit bewirkt eine Drehung des Dosiseinstellglieds 102 in die erste Drehrichtung eine Drehung des Dosisanzeigeglieds 110 ebenfalls in die erste Drehrichtung und eine Drehung des Dosiseinstellglieds 102 in die zweite Drehrichtung ebenfalls eine Drehung des Dosisanzeigeglieds 110 in die zweite Drehrichtung. Durch die Drehung des Dosiseinstellglieds 102 in die erste Drehrichtung wird das Dosisanzeigeglied 110 in die proximale Richtung geschraubt, wobei durch die Drehung des Dosiseinstellglieds 102 in die zweite Drehrichtung das Dosisanzeigeglied 110 in die distale Richtung geschraubt wird.

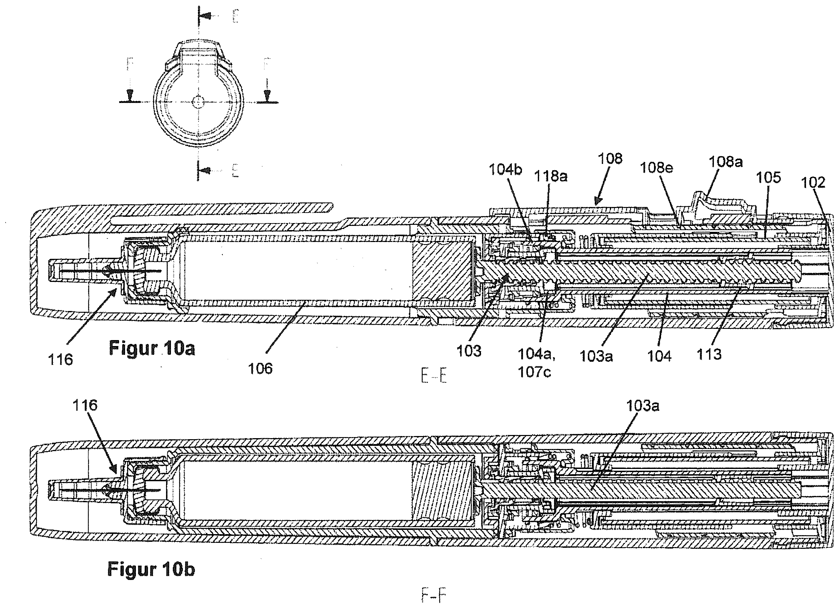

Das Betätigungsglied 108 weist einen um die Längsachse L gekrümmten und über eine Teil des Umfangs erstreckenden länglichen Hauptabschnitt 108c auf, der insbesondere außerhalb der Außenhülse 101a angeordnet ist und von dem ein Betätigungsabschnitt 108a nach außen abragt, um zum Beispiel von einem Finger des Benutzers der Vorrichtung verschoben werden zu können, um das Betätigungsglied 108 aus einer unbetätigten Position (Figur 8a) in die distale Richtung in eine betätigte Position (Figur 10a) verschieben zu können. Das Betätigungsglied 108, insbesondere dessen Hauptabschnitt 108c weist eine Zeigeeinrichtung 108d auf, die in dem gezeigten Beispiel als ein Fenster oder Durchbruch gebildet ist und über die eine aktuell eingestellte und auszuschüttende Produktdosis von der Dosisskala 110b abgelesen werden kann. Durch Verschieben des Betätigungsglieds 108 entlang der Längsachse L wird das Dosisanzeigeglied 110 aufgrund des Gewindeeingriffs entlang der Längsachse L mitgenommen.

Der Betätigungsabschnitt 108a ist proximal und entlang der Längsachse L in einer Flucht mit der Zeigeeinrichtung 108d angeordnet. Der Hauptabschnitt 108c greift an seinem distalen Ende durch einen Durchbruch der Außenhülse 101a in Umfangsrichtung unter die Außenhülse 101a und sichert somit das Betätigungsglied 108a an seinem distalen Ende gegen Herausfallen. Am proximalen Ende weist das Betätigungsglied 108 einen zungenförmigen Abschnitt auf, der die Außenhülse 10la und/oder das Dosiseinstellglied 102 an der Innenseite hintergreift, wodurch das proximale Ende des Betätigungsglieds 108 gegen Herausfallen gesichert ist.

Das Betätigungsglied 108 wird von einer Rücksetzfeder 109, die in dem gezeigten Beispiel als Wendelfeder gebildet ist und als Druckfeder entlang der Längsachse L wirkt, in die unbetätigten Position (Figur 8a) gedrückt. Durch die Betätigung des Betätigungsglieds 108 aus der unbetätigten in die betätigte Position wird die Feder 109 zusammengedrückt oder gespannt. Die Rücksetzfeder 109 stützt sich mit ihrem proximalen Ende an einem Koppelglied 115, insbesondere an einer Koppelhülse, ab, wobei das Koppelglied 115 das proximale Ende der Rücksetzfeder 109 axialfest mit dem Betätigungsglied 108 koppelt. Das Koppelglied 115 ist axialfest mit dem Betätigungsglied 108, insbesondere dem Hauptabschnitt 108c verbunden. Optional könnten das Betätigungsglied 108 und das Koppelglied 115 zusammen einteilig gebildet sein, wobei eine zweiteilige Ausgestaltung Montagevorteile bietet.

Das distale Ende der Rücksetzfeder 109 stützt sich insbesondere mittelbar, nämlich über das Kupplungsglied 118, das zum Beispiel als Kupplungsring ausgebildet sein kann, an dem Gehäuse 101, wie zum Beispiel an dem ersten Gehäuseeinsatz 101b axial ab.

Das ringförmige Kupplungsglied 118 weist an seinem distalen Ende eine stirnseitige Eingriffsstruktur 118b, insbesondere eine umlaufende Verzahnung auf, welche in eine Eingriffsgegenstruktur 101d, die am Gehäuse 101, insbesondere am ersten Gehäuseeinsatz 101b in proximale Richtung weisend angeordnet ist, eingreift. Die Eingriffsstrukturen 101d und 118b sind so geformt, dass für die Drehung des Kupplungsglieds 118 relativ zu dem Gehäuse 101 in die erste Drehrichtung am Kupplungsglied 118 ein geringeres Drehmoment erforderlich ist als für die Drehung in die zweite Drehrichtung. Dies kann dadurch erreicht werden, dass die in die erste Drehrichtung weisenden Zahnflanken der Eingriffsstruktur 118b und/oder in die zweite Drehrichtung weisenden Zahnflanken der Eingriffsstruktur 101d flacher sind als die in die zweite Drehrichtung weisenden Zahnflanken der Verzahnung 118b oder/und der in die erste Drehrichtung weisenden Zahnflanken der Eingriffsstruktur 101d.

Zwischen dem Zwischenglied 111 und dem Gehäuse 101 ist eine als Drehfeder wirkende und als Wendelfeder ausgestaltete Antriebsfeder 105 angeordnet. Das proximale Ende der Antriebsfeder 105 stützt sich an dem zweiten Gehäuseeinsatz 101c oder allgemein an dem Gehäuse 101 ab, wobei sich das distale Ende der Antriebsfeder 105 an dem Zwischenglied 111 abstützt. Durch Drehen des Dosiseinstellglieds 102 in die erste Drehrichtung wird die Antriebsfeder 105 gespannt und durch Drehen des Dosiseinstellglieds 102 in die zweite Drehrichtung wird die Antriebsfeder 105 entspannt.

Um zu verhindern, dass sich die Antriebsfeder 105 beim Loslassen des Dosiseinstellglieds 102 entspannt, sind die ineinandergreifenden Eingriffsstrukturen 101d und 118b vorgesehen. Das von der Antriebsfeder 105 auf das Kupplungsglied 118 wirkende Drehmoment ist, insbesondere auch in dem Fall, in dem die maximal einstellbare Dosis eingestellt ist, geringer als das Drehmoment, welches erforderlich ist, das Kupplungsglied 118 in die zweite Drehrichtung zu drehen. Dadurch wird sichergestellt, dass sich die Feder 105 nicht ungewollt entspannen kann. Das Haltemoment, welches erforderlich ist, das Kupplungsglied 118 relativ zu dem Gehäuse 101 in die erste Drehrichtung zu drehen, ist nicht nur von der Ausgestaltung der Eingriffsstruktur 101d und 118b, sondern auch von der Wahl der Federstärke und/oder der Federvorspannung der Rücksetzfeder 109 abhängig.

Das Kupplungsglied 118 weist an seinem Innenumfang eine dritte Kupplungsstruktur 118a auf, welche als Innenverzahnung gebildet ist. Das Antriebsglied 104 weist an seinem Außenumfang eine vierte Kupplungsstruktur 104b auf, die als Außenverzahnung gebildet ist und die in die dritte Kupplungsstruktur 118a eingreift, wenn das Betätigungsglied 108 in seiner unbetätigten Position ist. Das Kupplungsglied 118 und das Antriebsglied 104 greifen drehfest und axial verschiebbar ineinander, wenn das Betätigungsglied 108 in seiner unbetätitgten Position ist. Die dritte Kupplungsstruktur 118 und die vierte Kupplungsstruktur 104 bilden eine zweite Kupplung 104b, 118a.

Das Antriebsglied 104 weist eine erste Kupplungsstruktur 104a, welche als Innenverzahnung ausgestaltet ist, auf. Das Rotationsglied 107, insbesondere die Kupplungshülse 107a weist eine zweite Kupplungsstruktur 107c, welche als Außenverzahnung gebildet ist und in die erste Kupplungsstruktur 104a eingreift, wenn das Betätigungsglied 108 betätigt ist, und nicht eingreift, wenn das Betätigungsglied 108 nicht betätigt ist.

Die erste Kupplungsstruktur 104a und die zweite Kupplungsstruktur 107c bilden eine erste Kupplung 104a, 107c. Das Betätigungsglied 108 ist insbesondere über das Koppelglied 115 axialfest mit dem Antriebsglied 104 gekoppelt oder verbunden. Insbesondere greift das Koppelglied 115 axialfest und drehbar in das Antriebsglied 104 ein. Das Betätigungsglied 108 und das Koppelglied 115 sind in einem axialfesten, insbesondere auch verdrehfesten Eingriff.

Die Antriebs- und Dosiervorrichtung der zweiten Ausführungsform weist einen Mechanismus auf, welcher die Einstellung einer Dosis verhindert, welche die in dem Produktbehälter 106 enthaltene oder ausschüttbare Produktmenge übersteigt. Dieser Mechanismus umfasst ein hülsenförmiges Stoppelement 113, welches ein Innengewinde 113a aufweist, welches in das Außengewinde 103c der Gewindestange 103a eingreift. Das Stoppelement 113 umgebende Antriebsglied 104 und das Stoppelement 113 greifen verdrehfest und axial verschiebbar ineinander, wodurch das Stoppelement 113 die Drehungen des Antriebsglieds 104 mitmacht. Das Stoppelement 113 weist einen Stoppanschlag 113b auf, der an einen Stoppgegenanschlag 107e, der von dem Rotationsglied 107, insbesondere der Kupplungshülse 107a gebildet wird, anschlagen kann. Der sich entlang einer helixförmigen Kurve erstreckende Abstand zwischen dem Stoppanschlag 113b und dem Stoppgegenanschlag 107e ist proportional zu der in dem Produktbehälter 106 enthaltenen oder ausschüttbaren Produktmenge.

Das Dosisanzeigeglied 110 weist einen in Umfangsrichtung wirkenden Nulldosisanschlag 110d und einen in Umfangsrichtung wirkenden Maximaldosisanschlag 110c auf. Das Kopplungsglied 115 weist einen Nulldosisgegenanschlag 115a auf, gegen den der Nulldosisgegenanschlag 110d gedrückt wird, wenn versucht wird, eine Dosis einzustellen, die kleiner als null ist. Das Gehäuse 101, insbesondere der zweite Gehäuseeinsatz 101c weist einen Maximaldosisgegenanschlag 101e auf, gegen den der Maximaldosisanschlag 110c gedrückt wird, wenn versucht wird, eine Dosis einzustellen, welche über der maximal einstellbaren Dosis, wie zum Beispiel 80 IU, liegt. In der Nulldosisposition wird in der Zeigeeinrichtung 108d die Dosis Null angezeigt, wobei in der Maximaldosisposition in der Zeigeeinrichtung 108d die maximal mit der Vorrichtung einstellbare Produktdosis, hier 80 IU, angezeigt wird. Das Dosisanzeigeglied 110 ist zwischen der Nulldosisposition und der Maximaldosisposition hin und her schraubbar, insbesondere durch Hin- und Herdrehen des Dosiseinstellglieds 102 in die erste und zweite Drehrichtung.

Die Antriebs- und Dosiervorrichtung der zweiten Ausführungsform weist optional eine Einrichtung auf, die signalisiert, wenn das Betätigungsglied 108 betätigt ist (BetätigungsMarkierung) und/oder das Dosisanzeigeglied 110 in seiner Nulldosisposition ist (Nulldosis-Markierung) unabhängig von der Zeigeeinrichtung 108d, welche die aktuell eingestellte Dosis anzeigt. Für die Betätigungs-Markierung kann der Hauptabschnitt 108c sich des Betätigungsglieds 108 einen von dem Hauptabschnitt 108c erstreckenden Flügel (nicht dargestellt) aufweisen, an dem eine Markierung angeordnet sein kann. Alternativ kann der Flügel neben der Markierung einen transparenten Bereich (nicht gezeigt) aufweisen. In der unbetätigten Position des Dosiseinstellglieds 108 kann die Markierung so unterhalb eines von dem Gehäuse 101, insbesondere der Außenhülse 101a gebildeten Sichtfensters (nicht gezeigt) angeordnet sein, dass die Markierung von dem Benutzer über das Sichtfenster ablesbar wird. Durch das Verschieben des Betätigungsglieds 108 aus der unbetätigten Position in die betätigte Position kann die Betätigungs-Markierung zusammen mit dem Betätigungsglied 108 verschoben werden, wobei die Betätigungs-Markierung aus der Position unterhalb des Sichtfensters verschoben wird, so dass sie nicht mehr ablesbar ist. Dabei kann der transparente Bereich (nicht gezeigt) in das Sichtfenster verschoben werden und das Fenster insbesondere die Vorrichtung vor äusseren Eingriffen schützen. Die Betätigungs-Markierung wird von einem Abschnitt der Außenhülse 101a abgedeckt. Das Sichtfenster kann winkelversetzt und distal und/oderproximal des Betätigungsabschnitts 108a angeordnet sein, so dass der Benutzer der Vorrichtung bei Betätigung des Betätigungsabschnitts 108a das Sichtfenster möglichst nicht verdeckt. Hierdurch wird dem Benutzer zusätzlich ermöglicht, visuell festzustellen, ob das Betätigungsglied 108 betätigt ist.

Für eine Nulldosis-Markierung kann das Dosisanzeigeglied 110 eine von der Dosisanzeigeskala 110b abgesetzte Markierung (nicht gezeigt) aufweisen, welche in der Nulldosisposition des Dosisanzeigeglieds 110 unter dem Sichtfenster angeordnet ist, wodurch dem Benutzer der Vorrichtung bei betätigtem Betätigungsglied 108 visuell angezeigt werden kann, ob das Dosisanzeigeglied 110 in seiner Nulldosisposition ist, unabhängig von dem in der Zeigeeinrichtung 108d angezeigten Skalenwert. Durch das Verschieben des Betätigungsglieds 108 in die betätigte Position kann die Betätigungs-Markierung aus dem Sichtfenster verschoben werden, dabei kann der transparente Bereich der Betätigungs-Markierung in dem Sichtfenster erscheinen. Wenn eine Nulldosis eingestellt wurde oder wenn die Produktausschüttung erfolgt ist, wird in dem Sichtfenster, unterhalb des transparenten Bereichs, die Nulldosis-Markierung ablesbar, dabei wird die Nulldosis-Markierung von dem transparenten Bereich überlagert. Alternativ kann der transparente Bereich Pigmente oder Muster aufweisen, welche in der überlagerten Position mit der abgesetzten Markierung der Dosisanzeigeskala 110a zusammen eine Nulldosis-Markierung bilden. Hierdurch erhält der Benutzer eine Anzeige darüber, ob die Produktausschüttung vollständig beendet wurde. Alternativ kann die Markierung unterhalb einem Fenster des Dosiseinstellglieds 102 angeordnet sein, so dass bei einer Nulldosiseinstellung bzw. bei einer erfolgten Produktausschüttung in dem Fenster des Dosiseinstellglieds 102 die Nulldosis-Markierung erscheint und dem Benutzer mittgeteilt wird, dass die Produktausschüttung erfolgt ist.

In den Figuren 8a bis 8d werden verschiedene Ansichten der Injektionsvorrichtung in einem Ausgangs- oder Auslieferungszustand gezeigt. Zur Einstellung und Verabreichung einer Produktdosis wird die Kappe 114 abgenommen und eine Dosis durch Drehen des Dosiseinstellglieds 102 relativ zu dem Gehäuse eingestellt. Für die Dosiserhöhung wird das Dosiseinstellglied 102 in eine erste Drehrichtung gedreht, für eine Dosisverringerung in die zweite Drehrichtung. Während des Dosiseinstellens ist das Betätigungsglied 108 unbetätigt. In dem Sichtfenster (nicht gezeigt) kann optional die Betätigungs-Markierung angezeigt werden, wodurch hervorgeht, dass das Betätigungsglied 108 unbetätigt ist. Über die Zeigeeinrichtung 108d kann die aktuell eingestellte Produktdosis abgelesen werden. Bei unbetätigtem Betätigungsglied 108 ist die erste Kupplung 104a, 107c ausgekuppelt und die zweite Kupplung 104b, 118a eingekuppelt. Dadurch kann das Antriebsglied 104 relativ zu dem Rotationsglied 107 verdreht werden, ohne dass sich das Rotationsglied 107 mit dem Antriebsglied 104 mitdreht. Beim Erhöhen der Dosis, d.h. während des Drehens des Dosiseinstellglieds 102 in die erste Drehrichtung, wird die Antriebsfeder 105 gespannt und beim Verringern der Dosis, d.h. während des Drehens des Dosiseinstellglieds 102 in die zweite Drehrichtung, entspannt. Beim Verringern der Dosis rastet das Kupplungsglied 118 insbesondere die Verzahnung 118b über die Eingriffsstruktur 101d des ersten Gehäuseeinsatzes 101b in die zweite Drehrichtung, da zusätzlich zu dem Drehmoment der Antriebsfeder 105 das von dem Benutzer auf das Dosiseinstellglied 102 in die zweite Drehrichtung aufgebrachte Drehmoment auf das Kupplungsglied 118 wirkt.

Während der Dosiseinstellung, wenn sich das Antriebsglied 104 relativ zu dem Abtriebsglied 103 dreht, wird das Stoppelement 113, welches sich mit dem Antriebsglied 104 mitdreht, an der Gewindestange 103a entlang geschraubt. Bei der Drehung des Dosiseinstellglieds 102 in die erste Drehrichtung wird das Stoppelement 113 an der Gewindestange 103a in die distale Richtung geschraubt, wodurch sich der Abstand zwischen dem Stoppanschlag 113b und dem Stoppgegenanschlag 107e verringert. Wenn das Dosiseinstellglied 102 bei der Dosiskorrektur oder bei ausgekuppelter erster Kupplung 104a, 107c in die zweite Drehrichtung gedreht wird, wird das Stoppelement 113 in die proximale Richtung an der Gewindestande 103a entlang geschraubt, wodurch sich der Abstand zwischen dem Stoppanschlag 113b und dem Stoppgegenanschlag 107e vergrößert.

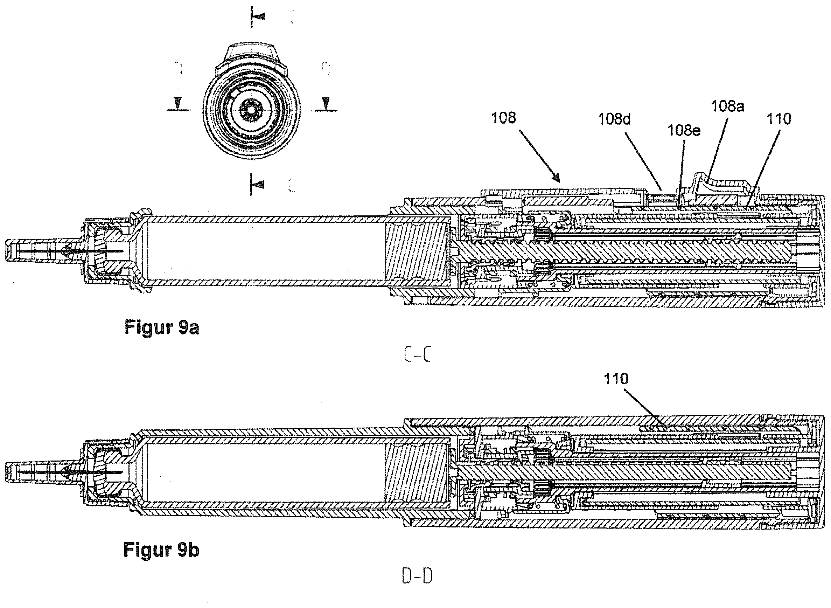

In den Figuren 9a und 9b wird die Injektionsvorrichtung in einem maximal aufdosierten Zustand gezeigt, wobei das Betätigungsglied 108 unbetätigt ist. Zum Ausschütten der eingestellten Produktdosis wird das Betätigungsglied 108 durch Betätigen des Betätigungsabschnitts 108a aus der unbetätigten Position in die distale Richtung in die betätigte Position verschoben, wodurch die Ausschüttung der eingestellten Dosis ausgelöst wird. Ferner kann durch das Verschieben des Betätigungsglieds 108 bewirkt werden, dass die Betätigungs-Markierung (nicht gezeigt) aus dem Sichtfenster verschoben wird und ein transparenter Bereich (nicht gezeigt) im Sichtfenster erscheint.

In den Figuren 10a und 10b wird die Injektionsvorrichtung, die sich in dem maximal aufdosierten Zustand befindet, mit einem betätigten Betätigungsglied 108 gezeigt. Durch die Betätigung des Betätigungsglieds 108 wird das Dosisanzeigeglied 110 aufgrund des Gewindeeingriffs von dem Betätigungsglied 108 mitgenommen, d. h. entlang der Längsachse L verschoben, zunächst noch ohne Drehbewegung. Die Verschiebung des Betätigungsglieds 108 in die betätigte Position bewirkt, dass das Koppelglied 115 von dem Betätigungsglied 108 mitgenommen wird, wodurch die Rücksetzfeder 109 zusammengedrückt wird. Wenn der Benutzer das Betätigungsglied 108 loslässt, setzt die Rücksetzfeder 109 das Betätigungsglied 108 aus der betätigten Position in die betätigte Position zurück.

Die Verschiebung des Betätigungsglieds 108 in die betätigte Position bewirkt über das Koppelglied 115 eine Mitnahme oder Verschiebung des Antriebsglieds 104 in die distale Richtung, wodurch zunächst die erste Kupplung 104a, 107c eingekuppelt und anschließend, d. h. erst wenn die erste Kupplung 104a, 107c eingekuppelt ist, die zweite Kupplung 104b, 118a ausgekuppelt wird. Durch die eingekuppelte erste Kupplung 104a, 107c findet eine Drehmomentübertragung von dem Antriebsglied 104 auf das Rotationsglied 107 statt. Durch das Auskuppeln der zweiten Kupplung 104b, 118a wird das Antriebsglied 104 für eine Rotation in die zweite Drehrichtung aufgrund des Drehmoments der gespannten Antriebsfeder 105 freigegeben, wodurch sich das Antriebsglied 104 in die zweite Drehrichtung dreht, wodurch das Rotationsglied 107 ebenfalls in die zweite Drehrichtung gedreht wird. Dadurch verschiebt sich das Abtriebsglied 103 in die distale Richtung und nimmt den Kolben im Produktbehälter 106 mit, wodurch das Produkt durch die Nadel der Nadeleinheit 116 ausgegeben wird. Während der Produktausschüttung bzw. Drehbewegung des Antriebsglieds 104 wird das Dosisanzeigeglied 110 zu dem Nulldosisgegenanschlag 115a des Koppelglieds 115 hingeschraubt und es werden von dem Ratschenglied 117 Klickgeräusche erzeugt.

Da das Abtriebsglied 103 und das Antriebsglied 104 sich während der Produktausschüttung, d. h. wenn die erste Kupplung 104a, 107e eingekuppelt ist, gemeinsam um die Längsachse drehen und relativ zueinander nicht drehen, behält das Stoppelement 113 seine Axialposition in Bezug auf das Antriebsglied 104 bei, wobei sich die Gewindestange 103a durch das Stoppelement 113 hindurch in distale Richtung schraubt. Der Abstand zwischen dem Stoppanschlag 113b und dem Stoppgegenanschlag 107e ändert sich während der Produktausschüttung nicht.

Die Ausschüttung der eingestellten Produktdosis ist beendet, wenn der Nulldosisanschlag 110d an den Nulldosisgegenanschlag 1 15a anschlägt. Wenn das Betätigungsglied 108 bis zum Ende der Produktausschüttung in der betätigten Position gehalten wird, kann die Nulldosis-Markierung (nicht gezeigt) in dem Sichtfenster (nicht gezeigt) erscheinen, wodurch das Ende der Produktausschüttung visuell angezeigt wird. Wenn der Betätigungsabschnitt 108a losgelassen wird, wird das Betätigungsglied 108 aufgrund der Rücksetzfeder 109 in die unbetätigte Position zurückgesetzt, wobei die Betätigungs-Markierung in dem Sichtfenster erscheint. Nun kann die Dosiseinstellung und die Dosisausschüttung wiederholt werden, vorausgesetzt es ist noch genügend Produkt in dem Produktbehälter 106.

Während der Produktausschüttung dreht sich das Dosiseinstellglied 102 in die zweite Drehrichtung mit. Optional könnte zwischen dem Antriebsglied 104 und dem Dosiseinstellglied 102 eine zusätzliche Kupplung (nicht gezeigt) vorgesehen sein, welche bei unbetätigtem Betätigungsglied 108 eingekuppelt und durch die Betätigung des Betätigungsglieds 108 ausgekuppelt wird, wodurch bewirkt, wird, dass sich das Dosiseinstellglied 102 während der Produktausschüttung nicht mit dem Antriebsglied 104 mitdreht. Weiter optional könnte noch eine zusätzliche Kupplung zwischen dem Dosiseinstellglied 102 und dem Gehäuse 101 oder einem gehäusefesten Element vorgesehen, sein, welche bei unbetätigtem Betätigungsglied 108 ausgekuppelt und bei betätigtem Betätigungsglied 108 eingekuppelt ist, wodurch das Dosiseinstellglied 102 während der Produktausschüttung relativ zu dem Gehäuse drehfest ist und bei der Dosiseinstellung relativ zu dem Gehäuse 101 drehbar ist.

Wenn die in dem Produktbehälter enthaltene Menge kleiner ist als die maximal einstellbare Dosis und versucht wird, eine Dosis einzustellen, die größer als die in dem Produktbehälter 106 enthaltene oder ausschüttbare Produktmenge ist, wird das Stoppelement 113 bei dem Versuch, das Dosiseinstellglied 102 in die erste Drehrichtung zu drehen, insbesondere der Stoppanschlag 113b gegen den in Umfangsrichtung wirkenden Stoppgegenanschlag 107e des Rotationsglieds 107 gedrückt. Da das Rotationsglied 107 aufgrund des Ratschenglieds 117 nicht in die erste Drehrichtung relativ zu dem Gehäuse 101 drehbar ist, wird eine Drehung des Antriebsglieds 104 und somit des Dosiseinstellglieds 102 in die erste Drehrichtung blockiert, so dass ein weiteres Aufdosieren nicht möglich ist, auch wenn mit der Injektionsvorrichtung grundsätzlich eine höhere Dosis einstellbar wäre.

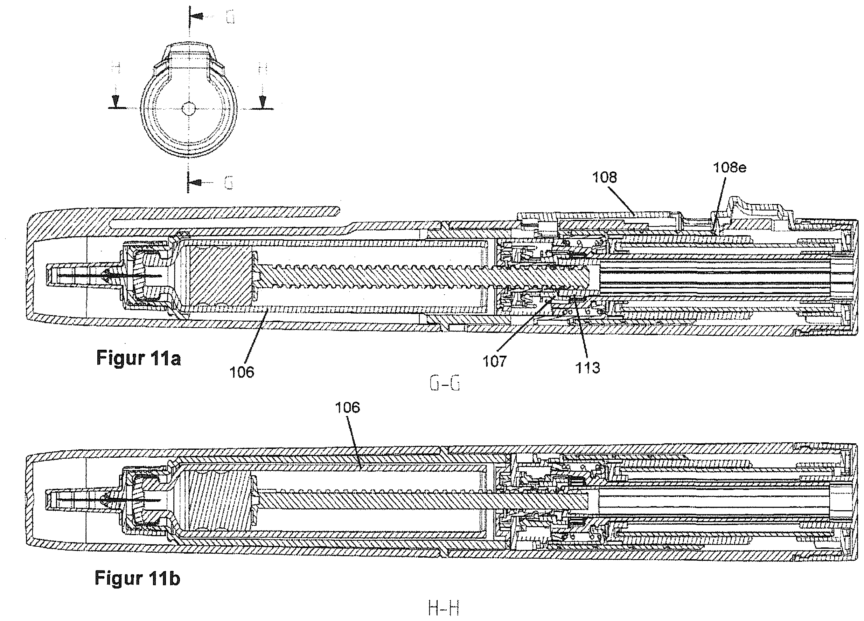

In den Figuren 11a und 11b wird die Injektionsvorrichtung am Ende der Ausschüttung der gesamten in dem Produktbehälter 106 enthaltenen Dosis gezeigt. Die Einstellung einer weiteren Dosis ist hier nicht mehr möglich, aufgrund des Stoppelements 113, welches an dem Rotationsglied 107 anschlägt.

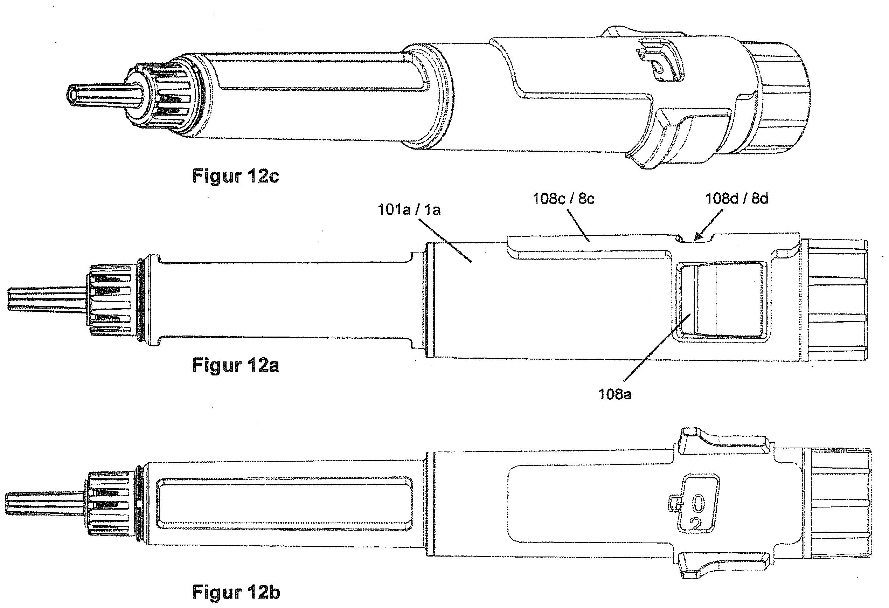

In den Figuren 12a und 12b wird eine Modifikation des Auslöseglieds 8; 108, insbesondere für die erste und zweite Ausführungsform, gezeigt. Der Übersichtlichkeit halber sind in der folgenden Beschreibung nur die Bezugszeichen der zweiten Ausführungsform angegeben, wobei die Figuren die Bezugszeichen für die erste und zweite Ausführungsform enthalten.

Das Auslöseglied 108 weist einen Hauptabschnitt 108c auf, der außerhalb der Außenhülse des Gehäuses 101 angeordnet ist. Von dem Hauptabschnitt 108c, welcher die Zeigeeinrichtung 108d, insbesondere das Fenster, aufweist, ragen in Umfangsrichtung beidseitig zwei Flügel ab, welche die Außenhülse 101a über ihren Außenumfang umgreifen, insbesondere den größten Teil des Umfangs umgreifen. Jeder der Flügel weist einen Betätigungsabschnitt 108a auf, der somit um die Längsachse L winkelversetzt zu der Zeigeeinrichtung 108d angeordnet sind. Durch diese Anordnung lässt sich das Betätigungsglied 108 mit dem Daumen und/oder dem Zeigefinger der die Außenhülse 101a umgreifenden Hand auf eine einfache Weise betätigen, ohne dass die Zeigeeinrichtung 108d verdeckt wird.

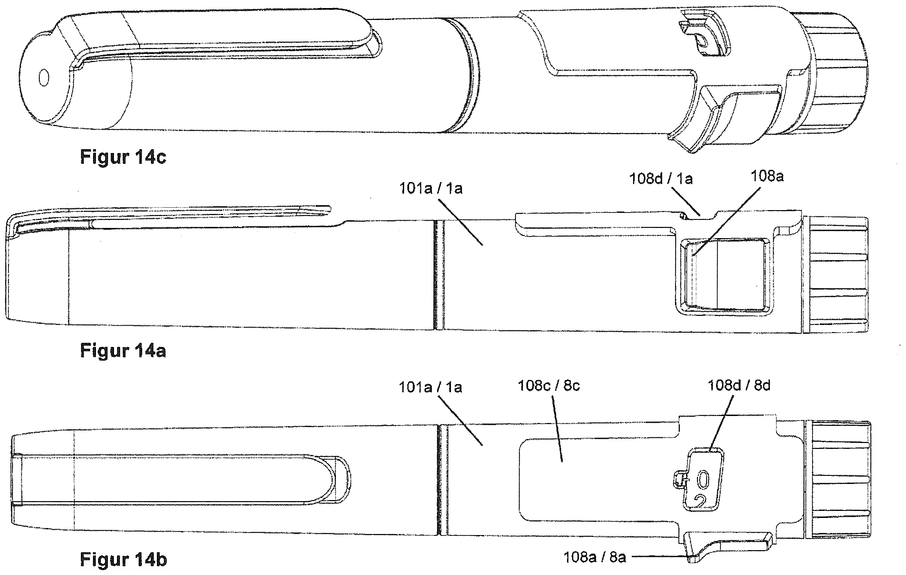

In den Figuren 14a und 14b wird eine Modifikation der Ausführung aus den Figuren 12a und 12b gezeigt, wobei nur einer der Flügel ein Auslöseglied 108a aufweist und der andere Flügel nicht. Bei dieser Ausführung lässt sich der Betätigungsabschnitt 108a zum Beispiel mit dem Daumen der die Außenhülse 101a umgreifenden Hand betätigen, ohne dass die Zeigeeinrichtung 108d verdeckt wird.

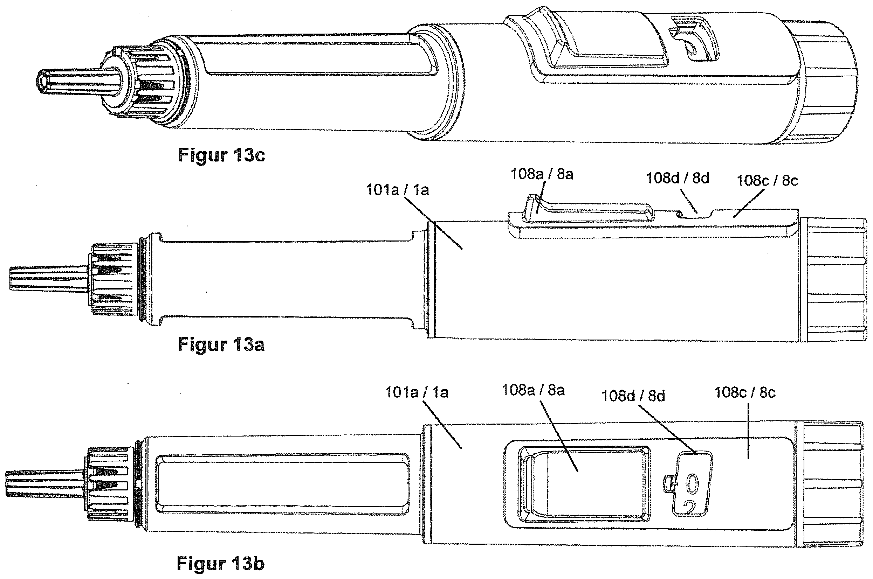

In den Figuren 13a und 13b wird eine weitere Modifikation des Betätigungsglieds 108 gezeigt, wobei der Hauptabschnitt 108c des Betätigungsglieds 108, der die Zeigeeinrichtung 108d, insbesondere das Fenster, aufweist, außerhalb der Außenhülse 101a, insbesondere an deren Außenumfang, angeordnet ist. Der Betätigungsabschnitt 108a ist distal der Zeigeeinrichtung und entlang der Längsachse L in einer Flucht mit der Zeigeeinrichtung 108d angeordnet. Dadurch kann der Betätigungsabschnitt 108a zum Beispiel mit dem Daumen der Hand, welche die Außenhülse 101a umgreift, betätigt werden, ohne dass die Zeigeeinrichtung 108d verdeckt wird.

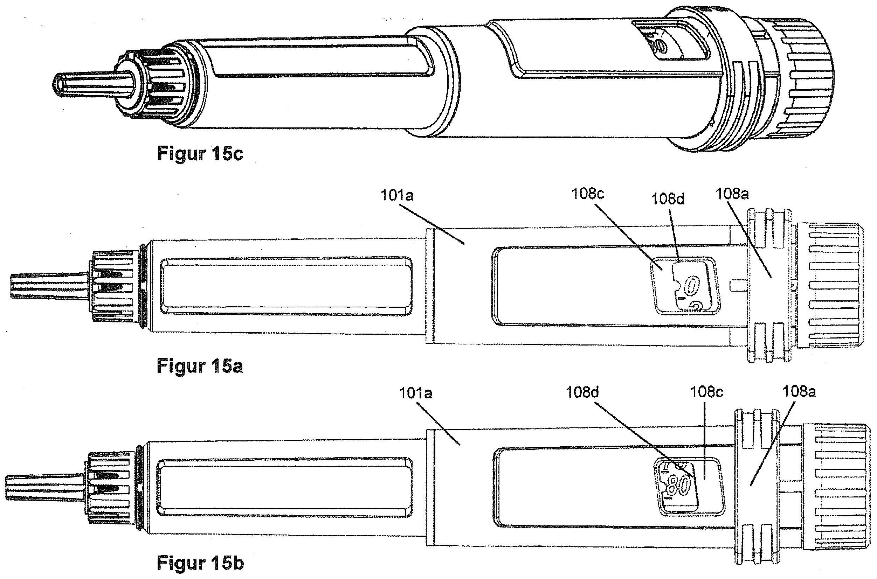

Die Figuren 15a und 15b zeigen eine weitere Modifikation des Betätigungsglieds 108, wobei der Hauptabschnitt 108c, der die Zeigeeinrichtung 108d aufweist, innerhalb der Außenhülse 10la, insbesondere an deren Innenumfang angeordnet ist. Das Betätigungselement 108 weist einen ringförmigen Betätigungsabschnitt 108a auf, welcher außerhalb der Außenhülse 101a angeordnet ist und die Außenhülse 101a über ihren Außenumfang umgibt. Der ringförmige Betätigungsabschnitt 108a ist mit dem Hauptabschnitt 108c über einen Verbindungssteg verbunden, welcher durch die Außenhülse 101a greift. Hierfür kann die Außenhülse 101a eine zum Beispiel schlitzförmige Ausnehmung aufweisen, durch welche sich der Steg erstreckt, der den ringförmigen Betätigungsabschnitt 108a und den Hauptabschnitt 108c, der die Zeigeeinrichtung 108d bildet, verbindet. Die schlitzförmige Ausnehmung kann gleichzeitig als Längsführung, d.h. auch als Verdrehsicherung, für das Betätigungsglied 108a dienen.

Der ringförmige Betätigungsabschnitt 108a lässt sich von dem Benutzer der Vorrichtung in verschiedenen Positionen greifen, so dass der Bedienkomfort erhöht wird und gleichzeitig verhindert werden kann, dass der Benutzer die Zeigeeinrichtung 108d mit einem Teil der Hand, welche die Außenhülse 101a umgreift, verdeckt. Der Betätigungsabschnitt 108a ist proximal der Zeigeeinrichtung 108d angeordnet, könnte aber distal der Zeigeeinrichtung 108d angeordnet sein.

Beispielsweise lässt sich der Betätigungsabschnitt 108a von dem Benutzer der Vorrichtung mit der Hand umgreifen, wobei er dann nicht mehr die Außenhülse 101a umgreift. Durch Andrücken der Injektionsvorrichtung an die Einstichstelle kann eine Push-on-Skin Auslösung realisiert werden, bei der die greifende Hand das Betätigungsglied 108, insbesondere den Betätigungsabschnitt 108a umgreift und in die distale Richtung verschiebt. Für diese Funktion kann der Betätigungsabschnitt 108a auch eine andere Gestalt, wie zum Beispiel eine hülsenförmige Gestalt aufweisen, so dass der Betätigungsabschnitt 108a noch besser greifbar ist.

**Bezugszeichenliste für die zweite Ausführungsform**

| | | | |
|---|---|---|---|
| 101 | Gehäuse | 109 | Rücksetzfeder/Klickfeder |
| 101a | Außenhülse | | |
| 101b | erster Gehäuseeinsatz | 110 | Dosisanzeige-glied/Dosisanzeigetrommel |
| 101c | zweiter Gehäuseeinsatz | | |
| 101d | Eingriffsgegenstruk- tur/Verzahnung | 110a | Außengewinde |
| | | 110b | Dosisskala |
| 101e | Maximaldosisgegenanschlag | 110c | Maximaldosisanschlag |
| | | 110d | Nulldosisanschlag |
| 102 | Dosiseinstellglied | | |
| | | 111 | Zwischenglied/Zwischenhülse |
| 103 | Abtriebsglied | | |
| 103a | Gewindestange | 112 | Produktbehälterhalter |
| 103b | Flansch | | |
| 103c | Außengewinde | 113 | Stoppelement/Stopphülse |
| 103d | Führungsnut | 113a | Innengewinde |
| | | 113b | Stoppanschlag |
| 104 | Antriebsglied | | |
| 104a | erste Kupplungsstruktur | 114 | Kappe |
| 104b | vierte Kupplungsstruktur | | |
| | | 115 | Koppelglied/Koppelhülse |
| 105 | Antriebsfeder | 115a | Nulldosisgegenanschlag |
| 106 | Produktbehälter/Karpule | 116 | Nadeleinheit |
| 107 | Rotationsglied | 117 | Ratschenglied/ unidirektionale Kupplung |
| 107a | Kupplungshülse | | |
| 107b | Gewindehülse | 117a | stirnseitige Verzahnung |
| 107c | zweite Kupplungsstruktur | 117b | Innenverzahnung |
| 107d | Innengewinde | 117c | Federabschnitt |
| 107e | Stoppgegenanschlag | | |
| 107f | Aussenverzahnung | 118 | Kupplungsglied |
| | | 118a | dritte Kupplungsstruktur |
| 108 | Betätigungsglied | 118b | Eingriffsstruktur/Verzahnung |
| 108a | Betätigungsabschnitt | | |
| 108c | Hauptabschnitt | 104a, 107c | erste Kupplung |
| 108d | Zeigeeinrichtung/Fenster | 104b, 118a | zweite Kupplung |
| 108e | Innengewinde | | |
| | | L | Längsachse |

## Patentansprüche

1. Antriebs- und Dosiervorrichtung für eine Injektionsvorrichtung zur Ausschüttung eines flüssigen Produkts, die Antriebs- und Dosiervorrichtung umfassend:
a) ein Gehäuse (1; 101),
b) ein Dosiseinstellglied (2; 102), welches für die Einstellung der auszuschüttenden Produktdosis relativ zu dem Gehäuse (1; 101) drehbar ist,
c) ein Betätigungsglied (8; 108), welches aus einer unbetätigten Position, die es während der Einstellung der auszuschüttenden Produktdosis einnimmt, in eine betätigte Position, die es während der Produktausschüttung einnimmt, entlang des Gehäuses axial verschiebbar ist, wobei das Verschieben des Betätigungsglieds (8; 108) in die betätigte Position eine Produktausschüttung auslöst und das Betätigungsglied (8; 108) einen Betätigungsabschnitt (8a; 108a) aufweist, auf den ein Benutzer der Antriebs- und Dosiervorrichtung Zugriff hat und mittels dem der Benutzer das Betätigungsglied (8; 108) aus der unbetätigten Position in die betätigte Position verschieben kann, wobei der Betätigungsabschnitt (8a; 108a) distal des Dosiseinstellglieds (2; 102) angeordnet ist, und
d) ein Dosisanzeigeglied (10; 110), wobei das Dosisanzeigeglied (10; 110) in der unbetätigten Position des Betätigungsglieds (8; 108) so mit dem Dosiseinstellglied (2; 102) gekoppelt ist, dass eine Drehung des Dosiseinstellglieds (2; 102) eine Drehung des Dosisanzeigeglieds (10; 110) bewirkt,
**dadurch gekennzeichnet, dass** das Betätigungsglied (8; 108) so mit dem Dosisanzeigeglied (10; 110) gekoppelt ist, dass das Dosisanzeigeglied (10; 110) die Bewegung des Betätigungsglieds (8; 108) mitmacht, wenn das Betätigungsglied (8; 108) aus der unbetätigten Position in die betätigte Position verschoben wird.

2. Antriebs- und Dosiervorrichtung nach Anspruch 1, **dadurch gekennzeichnet, dass** das Dosiseinstellglied (2; 102) das proximale Ende der Antriebs- und Dosiervorrichtung bildet.

3. Antriebs- und Dosiervorrichtung nach einem der Ansprüche 1 bis 2, **gekennzeichnet durch** eine von dem Betätigungsglied (8; 108) gebildete Zeigeeinrichtung (8d; 108d), wobei das Dosisanzeigeglied (10; 110) in der unbetätigten Position des Betätigungsglieds (8; 108) so mit dem Dosiseinstellglied (2; 102) gekoppelt ist, dass eine Drehung des Dosiseinstellglieds (2; 102) eine Drehung des Dosisanzeigeglieds (10; 110) bewirkt, wobei durch die Zeigeeinrichtung (8d; 108d) ein Skalenwert von einer am Außenumfang des Dosisanzeigeglieds (10; 110) angeordneten Skala (10b; 110b) ablesbar ist.

4. Antriebs- und Dosiervorrichtung nach einem der Ansprüche 1 bis 3, **dadurch gekennzeichnet, dass** das Betätigungsglied (8; 108) in Bezug auf das Gehäuse (1; 101) um die Längsachse (L) der Antriebs- und Dosiervorrichtung drehfest und entlang der Längsachse (L) verschiebbar ist.

5. Antriebs- und Dosiervorrichtung nach einem der Ansprüche 1 bis 4, **dadurch gekennzeichnet, dass** das Gehäuse (1) ein Sichtfenster (1e) und das Betätigungsglied (8) eine innerhalb des Gehäuses (1) angeordnete Markierung (8b) aufweist, wobei die Markierung (8b) so an dem Betätigungsglied (8) angeordnet ist, dass sie in der unbetätigten Position des Betätigungsglieds (8) in Bezug auf das Sichtfenster (1e) so positioniert ist, dass sie durch das Sichtfenster (1e) ablesbar ist, wobei die Markierung (8b) durch das Verschieben des Betätigungsglieds (8) in die betätigte Position in Bezug auf das Sichtfenster (1e) in eine Position verschoben wird, in der sie durch das Sichtfenster (1e) nicht mehr ablesbar ist.

6. Antriebs- und Dosiervorrichtung nach einem der Ansprüche 1 bis 5, **dadurch gekennzeichnet, dass** das Gehäuse (1, 101) ein Sichtfenster (1e) und das Dosisanzeigeglied (10, 110) eine innerhalb des Gehäuses (1, 101) angeordnete Nulldosis-Markierung (10e) aufweist, die sich insbesondere von der Skala (10b, 110b) unterscheidet, wobei die Nulldosis-Markierung (10e) so an dem Dosisanzeigeglied (10, 110) angeordnet ist, dass sie, wenn die eingestellte Dosis Null ist oder die eingestellte Dosis vollständig verabreicht wurde und, insbesondere wenn das Betätigungsglied (8, 108) in seiner betätigten Position ist, in Bezug auf das Sichtfenster (1e) so positioniert ist, dass sie durch das Sichtfenster (1e) ablesbar ist.

7. Antriebs- und Dosiervorrichtung nach einem der Ansprüche 1 bis 5, **dadurch gekennzeichnet, dass** die Antriebs- und Dosiervorrichtung ein Sichtfenster (le), das Betätigungsglied (8, 108) einen transparenten Bereich und das Dosisanzeigeglied (10, 110) eine von der Dosisanzeigeskala (10b, 110b) abgesetzte Markierung (10e) aufweist, wobei der transparente Bereich die abgesetzte Markierung (10e) überlagert und eine Nulldosis-Markierung bildet, welche, wenn die eingestellte Dosis Null ist oder die eingestellte Dosis vollständig verabreicht wurde und insbesondere das Betätigungsglied (8, 108) in seiner betätigten Position ist, durch das Sichtfenster (1e) ablesbar ist.

8. Antriebs- und Dosiervorrichtung nach einem der Ansprüche 1 bis 6, **dadurch gekennzeichnet, dass** das Gehäuse (1, 101) einen Durchbruch aufweist, wobei der Betätigungsabschnitt (8a, 108a) ein Vorsprung ist,
- der von einem innerhalb des Gehäuses (1, 101) angeordneten Hauptabschnitt (8c, 108c) des Betätigungsglieds (8, 108), der insbesondere die Zeigeeinrichtung (8d, 108d) bildet, ragt,
- der sich durch den Durchbruch erstreckt und
- dessen freies Ende über den Außenumfang des Gehäuses (1) ragt.

9. Antriebs- und Dosiervorrichtung nach einem der Ansprüche 1 bis 6, **dadurch gekennzeichnet, dass** das Gehäuse (1, 101) einen Durchbruch aufweist, wobei der Betätigungsabschnitt (8a, 108a) ein Vorsprung ist, der von einem außerhalb des Gehäuses (1, 101) angeordneten Hauptabschnitt (8c, 108c) des Betätigungsglieds (8, 108), der insbesondere die Zeigeeinrichtung (8d, 108d) bildet, nach außen ragt.

10. Antriebs- und Dosiervorrichtung nach Anspruch 7 oder 8, **dadurch gekennzeichnet, dass** der Betätigungsabschnitt (8a, 108a) proximal oder distal der Zeigeeinrichtung (8d, 108d) angeordnet ist.

11. Antriebs- und Dosiervorrichtung nach einem der vorhergehenden Ansprüche, **dadurch gekennzeichnet, dass** das Betätigungsglied (8, 108) das Gehäuse (1, 101) über dessen Umfang teilweise, wie zum Beispiel über den überwiegenden Teil oder vollständig, umgibt.

12. Antriebs- und Dosiervorrichtung nach einem der vorhergehenden Ansprüche **dadurch gekennzeichnet, dass** das Dosisanzeigeglied (10, 110) ein Aussengewinde (10a, 110a) aufweist und das Betätigungsglied (8, 108) mit einem Innengewinde (8e, 108e) in das Aussengewinde (10a, 110a) des Dosisanzeigeglieds (10, 110) eingreift, wobei das Dosisanzeigeglied (10, 110) an dem Betätigungsglied (8, 108) entlang schraubbar ist.

13. Antriebs- und Dosiervorrichtung nach einem der vorhergehenden Ansprüche, ferner umfassend
- ein in dem Gehäuse (1) aufgenommenes Abtriebsglied (3; 103),
- ein Antriebsglied (4; 104), welches relativ zu dem Gehäuse (1; 101) drehbar ist und während der Produktausschüttung so mit dem Abtriebsglied (3; 103) gekoppelt ist, dass eine Drehung des Antriebsglieds (4; 104) bewirkt, dass das Abtriebsglied (3; 103) relativ zu dem Gehäuse (1; 101) in die distale Richtung bewegt wird,
- eine Antriebsfeder (5; 105), welche das Antriebsglied (4; 104) drehend antreiben kann und die in ihr gespeicherte Energie als kinetische Energie an das Antriebsglied (4; 104) abgibt.

14. Antriebs- und Dosiervorrichtung nach einem der vorhergehenden Ansprüche, **gekennzeichnet durch** eine erste Kupplung (4a, 7a; 104a, 107c), welche zwischen dem Antriebsglied (4; 104) und dem Abtriebsglied (3; 103) angeordnet ist, wobei die erste Kupplung (4a, 7a; 104a, 107c) während der Einstellung der Produktdosis ausgekuppelt ist und hierdurch dem Antriebsglied (4; 104) erlaubt, relativ zu dem Abtriebsglied (3; 103) verdreht zu werden, wobei die erste Kupplung (4a, 7a; 104a; 107c) durch das Verschieben des Betätigungsglieds (8; 108) in seine betätigte Position eingekuppelt wird.

## Claims

1. Drive and metering apparatus for an injection apparatus for releasing a liquid product, the drive and metering apparatus comprising:
a) a housing (1; 101),
b) a dose setting member (2; 102) which is rotatable relative to the housing (1; 101) in order to set the product dose to be released,
c) an actuation member (8; 108) which is displaceable axially along the housing from a non-actuated position, which it assumes during the setting of the product dose to be released, to an actuated position, which it assumes during release of the product, wherein the displacement of the actuation member (8; 108) into the actuated position triggers a release of the product, and the actuation member (8; 108) has an actuation section (8a; 108a) which is accessible to a user of the drive and metering apparatus and by means of which the user can displace the actuation member (8; 108) from the non-actuated position to the actuated position, wherein the actuation section (8a; 108a) is arranged distal to the dose setting member (2; 102), and
d) a dose display member (10; 110), wherein, in the non-actuated position of the actuation member (8; 108), the dose display member (10; 110) is coupled to the dose setting member (2; 102) in such a way that a rotation of the dose setting member (2; 102) causes a rotation of the dose display member (10; 110),
**characterized in that** the actuation member (8; 108) is coupled to the dose display member (10; 110) in such a way that the dose display member (10; 110) follows the movement of the actuation member (8; 108) when the actuation member (8; 108) is displaced from the non-actuated position to the actuated position.

2. Drive and metering apparatus according to claim 1, **characterized in that** the dose setting member (2; 102) forms the proximal end of the drive and metering apparatus.

3. Drive and metering apparatus according to any one of claims 1 to 2, **characterized by** an indicator means (8d; 108d) formed by the actuation member (8; 108), wherein, in the non-actuated position of the actuation member (8; 108), the dose display member (10; 110) is coupled to the dose setting member (2; 102) in such a way that a rotation of the dose setting member (2; 102) causes a rotation of the dose display member (10; 110), wherein a scale value of a scale (10b; 110b) arranged on the outer circumference of the dose display member (10; 110) can be read by way of the indicator means (8d; 108d).

4. Drive and metering apparatus according to any one of claims 1 to 3, **characterized in that** the actuation member (8; 108) is rotationally fixed in relation to the housing (1; 101) about the longitudinal axis (L) of the drive and metering apparatus and is displaceable along the longitudinal axis (L).

5. Drive and metering apparatus according to any one of claims 1 to 4, **characterized in that** the housing (1) has a viewing window (1e), and the actuation member (8) has a marking (8b) arranged within the housing (1), wherein the marking (8b) is arranged on the actuation member (8) in such a way that, in the non-actuated position of the actuation member (8), the marking is positioned relative to the viewing window (1e) in such a way that it can be read through the viewing window (1e), wherein, as a result of the actuation member (8) being displaced into the actuated position, the marking (8b) is displaced relative to the viewing window (1e) into a position in which it can no longer be read through the viewing window (1e).

6. Drive and metering apparatus according to any one of claims 1 to 5, **characterized in that** the housing (1, 101) has a viewing window (1e), and the dose display member (10, 110) has a zero-dose marking (10e) which is arranged within the housing (1, 101) and which in particular differs from the scale (10b, 110b), wherein the zero-dose marking (10e) is arranged on the dose display member (10, 110) in such a way that, when the set dose is zero or the set dose has been fully administered, and in particular when the actuation member (8, 108) is in its actuated position, the zero-dose marking is positioned relative to the viewing window (1e) in such a way that it can be read through the viewing window (1e).

7. Drive and metering apparatus according to any one of claims 1 to 5, **characterized in that** the drive and metering apparatus has a viewing window (1e), the actuation member (8, 108) has a transparent region, and the dose display member (10, 110) has a marking (10e) which is set back from the dose display scale (10b, 110b), wherein the transparent region overlies the set-back marking (10e) and forms a zero-dose marking which, when the set dose is zero or the set dose has been fully administered and in particular the actuation member (8, 108) is in its actuated position, can be read through the viewing window (1e).

8. Drive and metering apparatus according to any one of claims 1 to 6, **characterized in that** the housing (1, 101) has an aperture, wherein the actuation section (8a, 108a) is a protrusion
- which protrudes from a main section (8c, 108c) of the actuation member (8, 108), which main section is arranged within the housing (1, 101) and in particular forms the indicator means (8d, 108d),
- which extends through the aperture and
- the free end of which protrudes beyond the outer circumference of the housing (1).

9. Drive and metering apparatus according to any one of claims 1 to 6, **characterized in that** the housing (1, 101) has an aperture, wherein the actuation section (8a, 108) is a protrusion which protrudes outward from a main section (8c, 108c) of the actuation member (8, 108), which main section is arranged outside the housing (1, 101) and in particular forms the indicator means (8d, 108d).

10. Drive and metering apparatus according to claim 7 or 8, **characterized in that** the actuation section (8a, 108a) is arranged proximal or distal to the indicator means (8d, 108d).

11. Drive and metering apparatus according to any one of the preceding claims, **characterized in that** the actuation member (8, 108) partially surrounds the housing (1, 101) around the circumference thereof, such as for example around the majority or entirely.

12. Drive and metering apparatus according to any one of the preceding claims, **characterized in that** the dose display member (10, 110) has an external thread (10a, 110a), and the actuation member (8, 108) engages with an internal thread (8e, 108e) in the external thread (10a, 110a) of the dose display member (10, 110), wherein the dose display member (10, 110) can be screwed along the actuation member (8, 108).

13. Drive and metering apparatus according to any one of the preceding claims, further comprising:
- a driven member (3; 103) which is accommodated in the housing (1),
- a drive member (4; 104) which is rotatable relative to the housing (1; 101) and during release of the product is coupled to the driven member (3; 103) in such a way that a rotation of the drive member (4; 104) causes the driven member (3; 103) to be moved relative to the housing (1; 101) in the distal direction,
a drive spring (5; 105) which is driven in rotation by the drive member (4; 104) and outputs its stored energy as kinetic energy to the drive member (4; 104).

14. Drive and metering apparatus according to any one of the preceding claims, **characterized by** a first coupling (4a, 7a; 104a, 107c) which is arranged between the drive member (4; 104) and the driven member (3; 103), wherein the first coupling (4a, 7a; 104a, 107c) is uncoupled during the setting of the product dose and as a result enables the drive member (4; 104) to be rotated relative to the driven member (3; 103), wherein the first coupling (4a, 7a; 104a, 107c) is coupled by displacing the actuation member (8; 108) into its actuated position.

## Revendications

1. Dispositif d'entraînement et de dosage pour un dispositif d'injection pour la distribution d'un produit liquide, le dispositif d'entraînement et de dosage comprenant :
a) un boîtier (1 ; 101),
b) un élément de réglage de dose (2 ; 102), lequel peut être amené en rotation par rapport au boîtier (1 ; 101) pour le réglage de la dose de produit à distribuer,
c) un élément d'actionnement (8 ; 108), lequel est déplaçable axialement le long du boîtier à partir d'une position non actionnée, qu'il occupe pendant le réglage de la dose de produit à distribuer, dans une position actionnée, qu'il occupe pendant la distribution de produit, dans lequel le déplacement de l'élément d'actionnement (8 ; 108) dans la position actionnée déclenche une distribution de produit et l'élément d'actionnement (8 ; 108) présente une partie d'actionnement (8a ; 108a), à laquelle un utilisateur du dispositif d'entraînement et de dosage a accès et au moyen de laquelle l'utilisateur peut déplacer l'élément d'actionnement (8 ; 108) à partir de la position non actionnée dans la position actionnée, dans lequel la partie d'actionnement (8a ; 108a) est disposée de manière distale par rapport à l'élément de réglage de dose (2 ; 102) ; et
d) un élément indicateur de dose (10 ; 110), dans lequel l'élément indicateur de dose (10 ; 110) dans la position non actionnée de l'élément d'actionnement (8 ; 108) est accouplé à l'élément de réglage de dose (2 ; 102), de sorte qu'une rotation de l'élément de réglage de dose (2 ; 102) provoque une rotation de l'élément indicateur de dose (10 ; 110) ;
**caractérisé en ce que** l'élément d'actionnement (8 ; 108) est accouplé à l'élément indicateur de dose (10 ; 110) de sorte que l'élément indicateur de dose (10 ; 110) suit le mouvement de l'élément d'actionnement (8 ; 108) lorsque l'élément d'actionnement (8 ; 108) est déplacé à partir de la position non actionnée dans la position actionnée.

2. Dispositif d'entraînement et de dosage selon la revendication 1, **caractérisé en ce que** l'élément de réglage de dose (2 ; 102) forme l'extrémité proximale du dispositif d'entraînement et de dosage.

3. Dispositif d'entraînement et de dosage selon l'une quelconque des revendications 1 à 2, **caractérisé par** un système indicateur (8d ; 108d) formé par l'élément d'actionnement (8 ; 108), dans lequel l'élément indicateur de dose (10 ; 110) dans la position non actionnée de l'élément d'actionnement (8 ; 108) est accouplé à l'élément de réglage de dose (2 ; 102), de sorte qu'une rotation de l'élément de réglage de dose (2 ; 102) provoque une rotation de l'élément indicateur de dose (10 ; 110), dans lequel une valeur d'échelle d'une échelle (10b ; 110b) disposée sur la périphérie extérieure de l'élément indicateur de dose (10 ; 110) peut être lue par le système indicateur (8d ; 108d).

4. Dispositif d'entraînement et de dosage selon l'une quelconque des revendications 1 à 3, **caractérisé en ce que** l'élément d'actionnement (8 ; 108) est déplaçable par rapport au boîtier (1 ; 101) de manière solidaire en rotation autour de l'axe longitudinal (L) du dispositif d'entraînement et de dosage et le long de l'axe longitudinal (L).

5. Dispositif d'entraînement et de dosage selon l'une quelconque des revendications 1 à 4, **caractérisé en ce que** le boîtier (1) présente une fenêtre d'inspection (1e) et l'élément d'actionnement (8) un marquage (8b) disposé à l'intérieur du boîtier (1), dans lequel le marquage (8b) est disposé sur l'élément d'actionnement (8), de sorte que dans la position non actionnée de l'élément d'actionnement (8), il est positionné par rapport à la fenêtre d'inspection (1e) de sorte qu'il peut être lu à travers la fenêtre d'inspection (1e), dans lequel le marquage (8b) du fait du déplacement de l'élément d'actionnement (8) dans la position actionnée est déplacé par rapport à la fenêtre d'inspection (1e) dans une position dans laquelle il ne peut plus être lu à travers la fenêtre d'inspection (1e).

6. Dispositif d'entraînement et de dosage selon l'une quelconque des revendications 1 à 5, **caractérisé en ce que** le boîtier (1, 101) présente une fenêtre d'inspection (1e) et l'élément indicateur de dose (10, 110) un marquage de dose nulle (10e) disposé à l'intérieur du boîtier (1, 101), qui se différencie en particulier de l'échelle (10b, 110b), dans lequel le marquage de dose nulle (10e) est disposé sur l'élément indicateur de dose (10, 110) de sorte que, lorsque la dose réglée est nulle ou la dose réglée a été entièrement administrée et, en particulier lorsque l'élément d'actionnement (8, 108) est dans sa position actionnée, il est positionné par rapport à la fenêtre d'inspection (1e) de sorte qu'il peut être lu à travers la fenêtre d'inspection (1e).

7. Dispositif d'entraînement et de dosage selon l'une quelconque des revendications 1 à 5, **caractérisé en ce que** le dispositif d'entraînement et de dosage présente une fenêtre d'inspection (1e), l'élément d'actionnement (8, 108) une zone transparente et l'élément indicateur de dose (10, 110) un marquage (10e) déposé par l'échelle indicatrice de dose (10b, 110b), dans lequel la zone transparente recouvre le marquage (10e) déposé et forme un marquage de dose nulle, lequel, lorsque la dose réglée est nulle ou la dose réglée a été entièrement administrée et en particulier l'élément d'actionnement (8, 108) est dans sa position actionnée, peut être lu à travers la fenêtre d'inspection (1e).

8. Dispositif d'entraînement et de dosage selon l'une quelconque des revendications 1 à 6, **caractérisé en ce que** le boîtier (1, 101) présente un passage, dans lequel la partie d'actionnement (8a, 108a) est une partie saillante,
- qui fait saillie d'une partie principale (8c, 108c) de l'élément d'actionnement (8, 108) disposée à l'intérieur du boîtier (1, 101), qui forme en particulier le système indicateur (8b, 108b),
- qui s'étend à travers le passage et
- dont l'extrémité libre fait saillie de la périphérie extérieure du boîtier (1).

9. Dispositif d'entraînement et de dosage selon l'une quelconque des revendications 1 à 6, **caractérisé en ce que** le boîtier (1, 101) présente un passage, dans lequel la partie d'actionnement (8a, 108a) est une partie saillante, qui fait saillie vers l'extérieur à partir d'une partie principale (8c, 108c) de l'élément d'actionnement (8, 108) disposée à l'extérieur du boîtier (1, 101), qui forme en particulier le système indicateur (8d, 108d).

10. Dispositif d'entraînement et de dosage selon la revendication 7 ou 8, **caractérisé en ce que** la partie d'actionnement (8a, 108a) est disposée de manière proximale ou distale par rapport au système indicateur (8d, 108d).

11. Dispositif d'entraînement et de dosage selon l'une quelconque des revendications précédentes, **caractérisé en ce que** l'élément d'actionnement (8, 108) entoure le boîtier (1, 101) en partie sur la périphérie de celui-ci, comme par exemple sur la majeure partie ou entièrement.

12. Dispositif d'entraînement et de dosage selon l'une quelconque des revendications précédentes, **caractérisé en ce que** l'élément indicateur de dose (10, 110) présente un filetage mâle (10a, 110a) et l'élément d'actionnement (8, 108) s'insère avec un filetage femelle (8e, 108e) dans le filetage mâle (10a, 110a) de l'élément indicateur de dose (10, 110), dans lequel l'élément indicateur de dose (10, 110) peut être vissé le long de l'élément d'actionnement (8, 108).

13. Dispositif d'entraînement et de dosage selon l'une quelconque des revendications précédentes, comprenant en outre :
- un élément de sortie (3 ; 103) logé dans le boîtier (1),
- un élément d'entraînement (4 ; 104), lequel peut être amené en rotation par rapport au boîtier (1 ; 101) et est accouplé à l'élément de sortie (3 ; 103) pendant la distribution de produit, de sorte qu'une rotation de l'élément d'entraînement (4 ; 104) a pour effet que l'élément de sortie (3 ; 103) est déplacé dans la direction distale par rapport au boîtier (1 ; 101),
- un ressort d'entraînement (5 ; 105), lequel peut entraîner l'élément d'entraînement (4 ; 104) en rotation et qui délivre l'énergie stockée dans celui-ci en tant qu'énergie cinétique à l'élément d'entraînement (4 ; 104).

14. Dispositif d'entraînement et de dosage selon l'une quelconque des revendications précédentes, **caractérisé par** un premier accouplement (4a, 7a ; 104a, 107c), lequel est disposé entre l'élément d'entraînement (4 ; 104) et l'élément de sortie (3 ; 103), dans lequel le premier accouplement (4a, 7a ; 104a, 107c) est désaccouplé pendant le réglage de la dose de produit et permet ainsi à l'élément d'entraînement (4 ; 104) d'être amené en rotation par rapport à l'élément de sortie (3 ; 103), dans lequel le premier accouplement (4a, 7a ; 104a ; 107c) est accouplé dans sa position actionnée par le déplacement de l'élément d'actionnement (8 ; 108).
